# EUROPEAN PATENT SPECIFICATION

(11) **EP 1 437 349 B1**
(45) Date of publication and mention of the grant of the patent: **15.08.2007**
(21) Application number: 02748888.1
(22) Date of filing: 17.07.2002
(51) Int. Cl.: C07D 261/08, A61K 31/42, A61P 31/00, A61P 35/00

(54) **SUBSTITUTED ISOXAZOLES AND THE USE THEREOF AS ANTIBIOTICS**
SUBSTITUIERTE ISOXAZOLE UND DEREN VERWENDUNG ALS ANTIBIOTIKA
ISOXAZOLES SUBSTITUES ET UTILISATION COMME ANTIBIOTIQUES

(30) Priority: 20.07.2001 ES 200101793
(43) Date of publication of application: 14.07.2004
(73) Proprietor: LABORATORIOS S.A.L.V.A.T., S.A., 08950 Esplugues de Llobregat (Barcelona) (ES)
(72) Inventor: FARRERONS GALLEMI, Carles, E-08034 Mataro (ES); LAGUNAS ARNAL, Carmen, E-08903 L'Hospitalet de Llobregat (ES); FERNANDEZ SERRAT, Anna, E-08190 Sant Cugat del Valles (ES); CATENA RUIZ, Juan Lorenzo, E-08901 L'Hospitalet de Llobregat (ES); MIQUEL BONO, Ignacio José, E-08902 L'Hospitalet de Llobregat (ES); BALSA LOPEZ, Dolors, E-08912 Badalona (ES); SALCEDO ROCA, Carolina, E-08757 Corbera (ES); TOLEDO MESA, Natividad, E-08410 Vilanova Del Vallès (ES); FERNANDEZ GARCIA, Andrés, E-08034 Barcelona (ES)
(74) Representative: Rambelli, Paolo
(86) International application number: PCT/ES2002/000358
(87) International publication number: WO 2003/008395

(56) References cited:
- WO-A-00/10566
- WO-A-01/40222
- WO-A-99/41244

## Description

The present invention relates to compounds with antibacterial activity containing a substituted isoxazole ring, as well as to processes for their preparation, to intermediates useful in their preparation and to pharmaceutical compositions containing them.

### BACKGROUND OF THE ART

The international microbiological community continues to express serious concern in view of the alarming increase of resistance to commercially available antibiotics, which reduces the range of possibilities of treatment of the different infectious processes. In general, bacterial pathogens may be classified as either Gram-positive or Gram-negative pathogens. Antibiotic compounds with effective activity against both Gram-positive and Gram-negative pathogens are generally regarded as having a broad spectrum of activity. The compounds of the present invention exhibit activity against both Gram-positive and Gram-negative pathogens.

Gram-positive pathogens, for example staphylococci, enterococci, and streptococci, are particularly important because of the development of the resistant strains which are both difficult to treat and difficult to eradicate from the hospital environment. Examples of such strains are methicillin resistant staphylococci, methicillin resistant coagulase negative staphylococci, penicillin resistant *Streptococcus pneumoniae* and multiply vancomycin resistant enterococci.

The best clinically effective antibiotic for treatment of such resistant Gram-positive pathogens is vancomycin. Vancomycin is a glycopeptide and is associated with nephrotoxicity an ototoxicity. Nevertheless, antibacterial resistance to vancomycin and other glycopeptides is also appearing and this resistance is increasing, rendering these agents less and less effective in the treatment of infections produced by Gram-positive pathogens.

Between 1989 and 1992, certain antibacterial compounds containing an oxazolidinone ring with a 5-acetamidomethyl side chain have been described (see for example, W.A. Gregory et al., J. Med. Chem. 1989, 32, 1673-1681; W.A. Gregory et al., J. Med. Chem. 1990, 33, 2569-2578; Chung-Ho Park et al. J. Med. Chem. 1992, 35, 1156-1165). Some examples of this compounds are DuP 105 and DuP 721, which reached the clinical state of development.

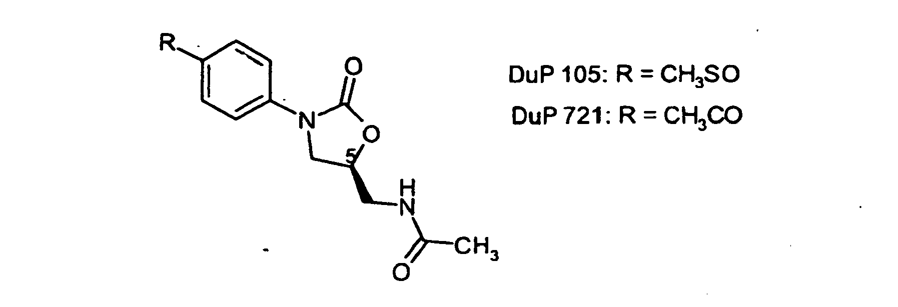

Afterwards, different modifications of certain substituents of the oxazolidinonic structure were made, rendering several compounds from which especially notable are U-100592 (eperezolid) and U-100766 (linezolid), both from Pharmacia Corporation (see for example, S.J. Brickner et al., J. Med. Chem. 1996, 39, 673-679). From them, only linezolid is commercially available at the moment.

Nevertheless, though the discovery of the mentioned oxazolidinones means a clear advance in the treatment of infections produced by Gram-positive pathogens, it is as well to remember that bacterial resistance to known antibacterial agents may develop, for example, by the evolution of active binding sites in the bacteria rendering a decrease or total loss of activity of pharmacophore previously active. Therefore, it is useful to obtain new antibacterial agents with other pharmacophores different to the ones containing an oxazolidinone ring.

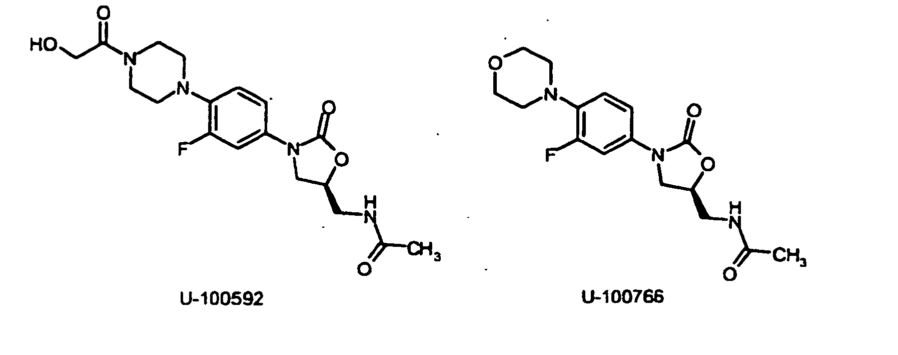

In this respect, Pharmacia Corporation has described the use of an isoxazoline central ring to obtain compounds with antibacterial activity (cf. WO 9807708, WO 9941244 and WO 9943671), which can be described by the following general structure:

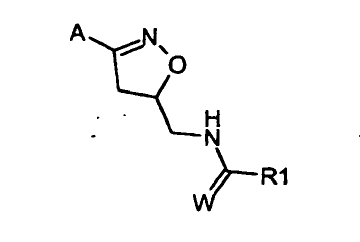

Bayer has described (cf. DE 19909785 A1) the use of an isoxazoline ring in compounds which have an A ring consisting of two fused rings.

AstraZeneca has described (cf. WO 01/40222 A1) the use of an isoxazoline central ring for the preparation of compounds with antibacterial activity with the following general structure:

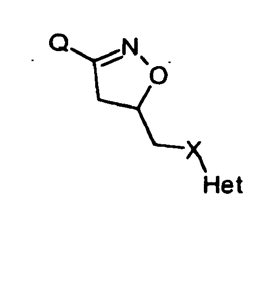

The aforementioned compounds have, at least, a chiral center in the carbon 5 of the isoxazoline ring. Nevertheless, it has been observed that only compounds with *R* configuration have antibacterial activity, what represents a drawback under a synthetic point of view.

On the other hand, Bristol-Myers Squibb (cf. WO 00/10566 A1) describes how to obtain antibacterial compounds containing an isoxazolinone central ring without any chiral center, with the following general structure:

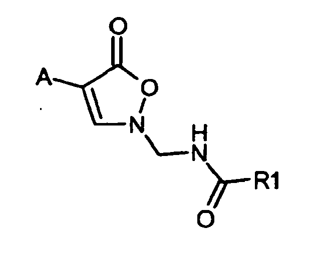

In the light of the background art, it is obvious the present interest in providing new compounds with antibacterial activity against both Gram-positive and Gram-negative pathogens, especially if they do not present chirality in the five membered ring.

### SUMMARY OF THE INVENTION

An aspect of the present invention relates to the provision of new (3,5)-disubstituted isoxazolinic type compounds of formula (I), 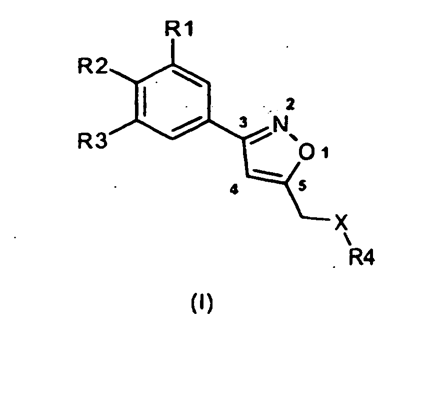 and stereoisomers, mixtures of stereoisomers, polymorphic forms, mixtures of polymorphic forms, *N*-oxides, solvates and pharmaceutically acceptable salts thereof, wherein
X is a biradical selected from the group consisting of O, S, NH, OCO, NH-CO, NH-COO, NH-CS, NH-CO-NH and NH-CS-NH;
R4 is a radical selected from the group consisting of hydrogen, a straight or branched (C₁-C₃)-alkyl, optionally substituted in any of their carbon atoms by 1, 2 or 3 atoms of F, Cl or Br; and a C-linked heterocyclic radical HET1 that is:
either a C-linked radical of a 5-membered heterocycle of 1, 2, 3 or 4 heteroatoms selected from the group consisting of N, O and S, optionally substituted by a radical selected from the group consisting of (C₁-C₄)-alkyl, amino, (C₁-C₄)-alkylamino, (C₁-C₄)-alkoxyl, (C₁-C₄)-alkoxycarbonyl, (C₁-C₄-alkylcarbonyl, (C₁-C₄)-amido, amido, CN, NO₂, F, Cl, and Br; or
a C-linked radical of a 6-membered heterocycle with 1, 2 or 3 atoms of nitrogen, optionally substituted by 1, 2 or 3 substituents independently selected from the group consisting of (C₁-C₄)-alkyl, amino, (C₁-C₄)-alkylamino, (C₁-C₄)-alkoxyl, (C₁-C₄)-alkoxycarbonyl, (C₁-C₄)-alkylcarbonyl, (C₁-C₄)-amido, amido, CN, NO₂, F, Cl and Br;
R1 and R3 each independently represent H or F;
R2 is an *N*-linked or *C*-linked radical selected from the following group: 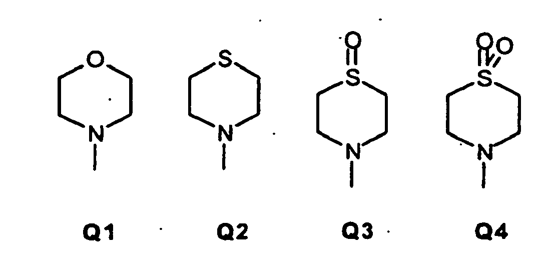 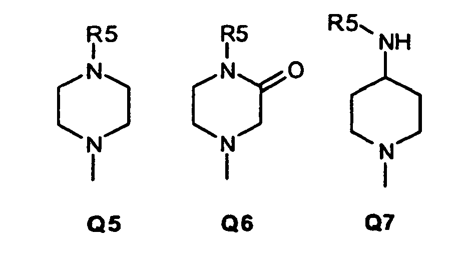 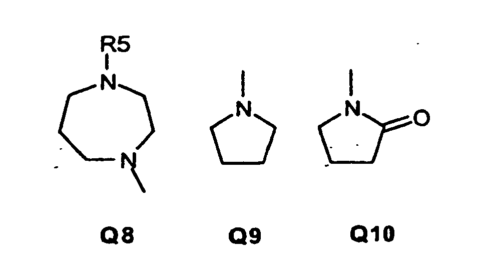 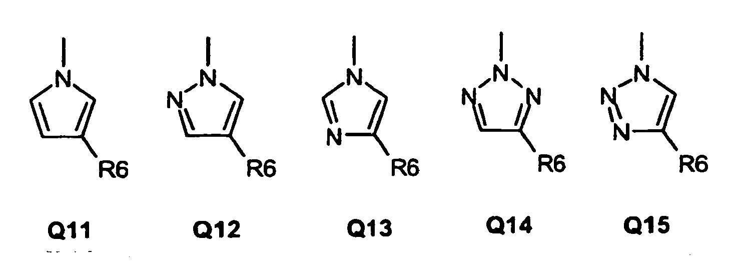 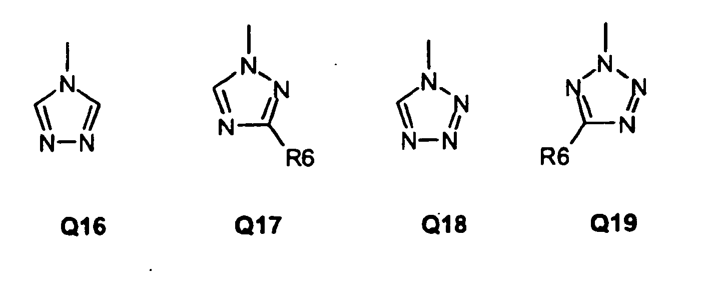 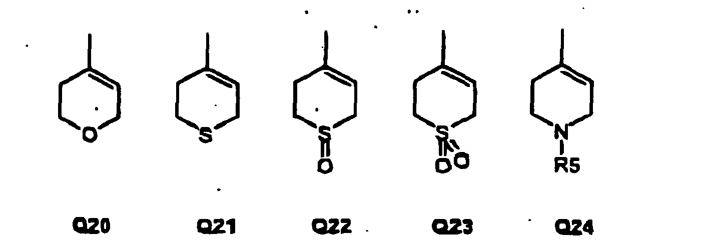 wherein:
R5 is a non cyclic radical selected from the group consisting of:
   -(CH₂)ₙ-CO-R7,
   and SO₂-R7
wherein:
R7 is (C₁-C₄)-alkyl, (C₁-C₃)-alkenyl (straight or branched), -(CH₂)ₚ-R2. -(CH₂)ₘ-Y-(CH₂)ₙ -R8 or HET2;
n, p, q and m are integers from 0 to 8;
Y is O, S or NH;
R2 is as defined above, excluding Q20, Q21, Q22, Q23 and Q24;
R8 is H or a C-linked radical selected from (C₁-C₃)-alkyl, vinyl, allyl, ethynyl, propargyl, phenyl phenyl substituted by CHO, CN, NO₂, CH₃ or F and a C-linked radical of an aromatic system constituted by a 5- or 6-membered ring, or by two 5- or 6-membered fused rings; containing the aforementioned aromatic system from one to three heteroatoms independently selected from O, N and S; and being the aforementioned aromatic system optionally mono-, di- or trisubstituted by radicals independently selected from the group consisting of H, (C₁-C₄)-alkyl (straight or branched), (C₁-C₄)-alkoxyl, (C₁-C₄)-alkylsulfanyl, NHCO-R9, NHCOO-R9, CO-R9, COO-R9, CN, NO, NO₂ CH=N-R10, F, Cl and Br or said aromatic system being selected from the group consisting of 2-, 3- and 4-pyridyl all of which may optionally be substituted by CHO, CN, NO₂, CH₃ or F
R9 is H, (C₁-C₃)-alkyl or N(R11)(R12), whereinR1 and R12 are independently selected from the group consisting of H and (C₁-C₂)-alkyl;
R10 is H, (C₁-C₃)-alkyl, phenyl, benzyl, OH or (C₁-C₃)-alkyloxy: HET2 is a C-linked heterocyclic radical selected from the group consisting of: 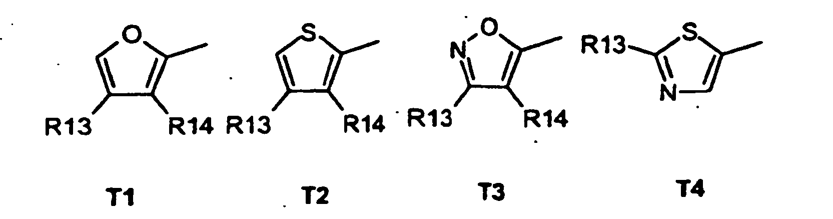 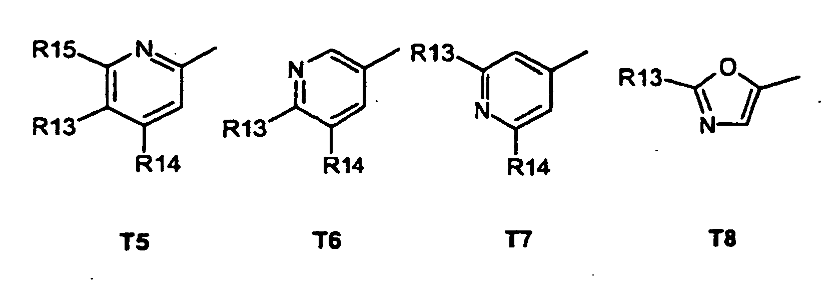 wherein R13, R14 and R15 are radicals independently selected from the group consisting of H, (C₁-C₄)-alkyl (straight or branched), (C₁-C₄)-alkoxyl, (C₁-C₄)-alkylsulfanyl, NHCO-R9, NHCOO-R9, CO-R9, COO-R9, CN, NO, NO₂, CH=N-R10, F, Cl and Br, wherein R9 and R10 are as defined above;
   alternatively, R5 is C-linked heterocyclic radical selected from the group consisting of: 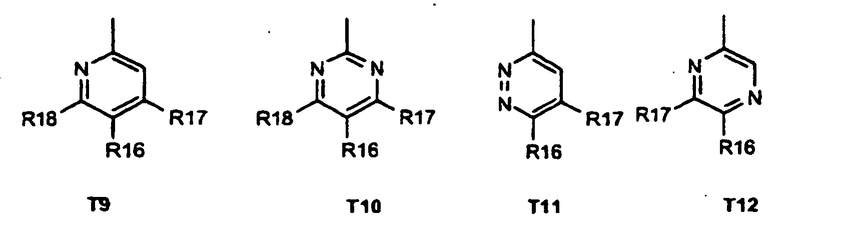 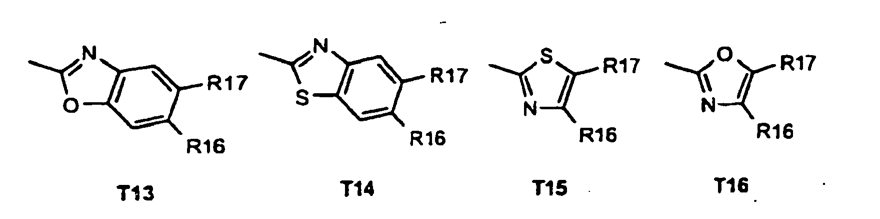 wherein R16, R17 and R18 are independently selected from the group consisting of CO-R9, COO-R9, CN, NO, NO₂, and CH=N-R10;
R6 is selected from the group consisting of H, F, Cl, Br, trifluoromethyl, CN, NO₂, CHO, CH₂OH, (C₁-C₃)alkyl, (C₁-C₃)-alkoxyl, (C₁-C₃)-alkoxycarbonyl, (C₁-C₃)-alkoxy-(C₁-C₃)-alkyl, benzyloxy-(C₁-C₃)-alkyl, (C₁-C₃)-yalkylcarbonyl, CO-NR19R20, NR19R20, (C₁-C₃)-alkylamino, (C₁-C₃)-alkyl-CH=N-O-R21, CH=N-O-R21, CH=CR22R23, (CH₂)ₛNHR19, and CH=NR19; wherein R19 and R20, are independently selected from the group consisting of H, (C₁-C₃)-alkyl, CO-R24 and an aromatic system constituted by a 5- or 6-membered ring, or by two 5- or 6-membered fused rings; optional containing the aforementioned aromatic system from one to three heteroatoms independently selected from O, N and S; and being the aforementioned aromatic system optionally mono-, di- or trisubstituted by radicals independently selected from the group consisting of H, (C₁-C₄)-alkyl (straight or branched), (C₁-C₄)-alkoxyl, (C₁-C₄)-alkylsulfanyl, NHCO-R9, NHCOO-R9, CO-R9, COO-R9, CN, NO, NO₂, CH=N-R10, F, Cl and Br; R21 is H or (C₁-C₃)-alkyl; R22 and R23, are independently selected from the group consisting of H, CN, NO₂, (C₁-C₃)-alkylcarbonyl, (C₁-C₃)-alkoxycarbonyl, CHO, CONR19R20 and CH₂OH; and R24 is H, (C₁-C₃)-alkyl, (C₁-C₃)-alkoxyl or HET2, wherein HET2 is as defined above; s is a integer comprised from 0 to 4.

Pharmaceutically acceptable salts include acid addition salts, such as mesilates, fumarates, hydrochlorides, citrates, maleates and tartrates. Also physiologically acceptable are salts formed with phosphoric and sulfuric acids. Likewise, suitable salts are basic salts, such as an alkaline metal salt, for example sodium, or an alkaline earth metal salt, for example calcium or magnesium. There may be more than one cation or anion depending on the number of charged functions and the valency of the cations or anions.

Some compounds of the formula (I) of the present invention may have one or several chiral centres. The present invention includes each of the stereoisomers, and racemic mixtures thereof. Optically active compounds can be prepared by commonly used processes, for example by resolution of the racemic mixture by recrystallisation techniques, by chiral synthesis, by enzymatic resolution, by biotransformation or by chromatographic resolution.

Certain compounds of the formula (I) of the present invention can exist in unsolvated as well as solvated forms such as, for example, hydrated forms. The present invention encompasses all such aforementioned forms which are pharmaceutically active.

Some compounds of the general formula (I) may exist as *N*-oxides of any of the oxidizable nitrogens of the mentioned compounds, encompassing the instant invention all *N*-oxides of the described compounds.

Certain compound of the general formula (I) may exhibit polymorphism, encompassing the present invention all the possible polymorphic forms.

In a particular embodiment, compounds of the present invention are those of formula (I) wherein:
**X is NH or NH-CS;**
R4 is methyl or a C-linked isoxazole or isothiazole radical optionally substituted by a methyl moiety in any of their substitutable positions;
R1 is H and R3 is F;
R2 is a radical selected from the following group: 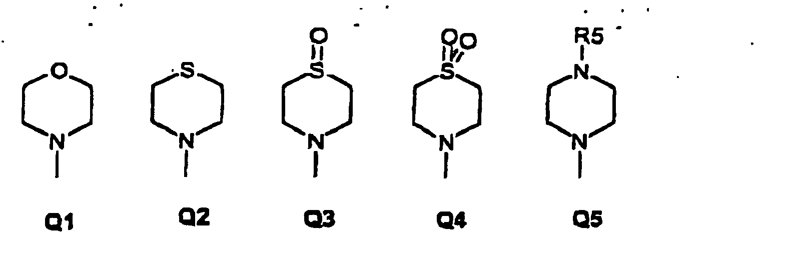 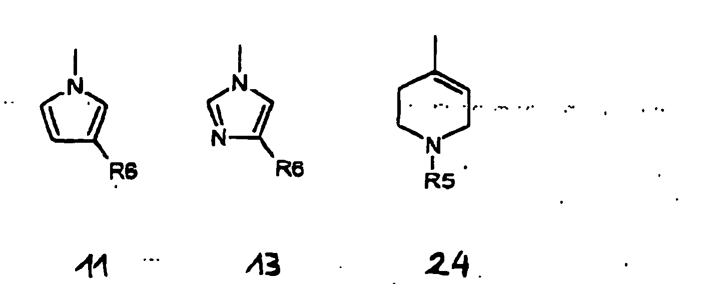
R5 is CO-R7;
R7 is selected from (CH₂)ₘ-Y-R8 and HET2, wherein m =1;
Y is O or NH;
R8 is selected from the group consisting of H, phenyl and 2-, 3-, 4-pyridyl, being the last four optionally substituted by CHO, CN, NO₂, CH₃ or F;
HET2 is selected from the group consisting of: 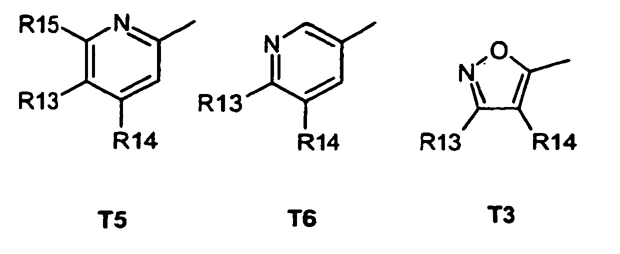 wherein R13, R14 and R15 are independently selected from the group consisting of CN, NO₂ and CHO;
and R6 is selected from the group consisting of H, CH₃, CN, CHO, CH₂OH, CH=N-OH, CH=CHCN, CO-CH₃ and CH₂NH-phenyl, said phenyl being substituted by a radical selected from the group consisting of F, CN, CHO and NO₂.

Subsequently, processes used for the preparation of the compounds of general structure (I), or for the preparation of pharmaceutically acceptable salts or *in vivo* hydrolyzable esters are described. The following schemes illustrate the processes carried out.

In Scheme 1, the process of synthesis of structures with a methanosulfonylmethylen radical in C5 is illustrated, said structures being used as precursors of several subclasses of final compounds.
By reaction of the conveniently substituted 4-fluorobenzaldehyde **1** with hydroxylamine hydrochloride, aldoxyme **2** is obtained. Aldoxyme **2** is reacted with *N*-chlorosuccinimide to give the corresponding *N*-hydroxybenzimidoyl chloride **3**, from which the nitrile oxide is formed *in situ* by reaction with triethylamine. Isoxazolic structure **4** is obtaining by a 1,3-dipolar cycloaddition type reaction between propargyl alcohol and the mentioned nitrile oxide to give an hydroxymethyl radical in C5 which is subsequently derivatized to the mesilate **5**.

In Scheme 2 the process of synthesis used to obtain the precursors with a methylencarbamate **8,** amido **7,** thiourea **9** or thioamide **10** radical in C5 is illustrated. In all cases, treatment of mesilate **5** with an ammonia alcoholic solution gives the aminomethylenic compound **6**. Carbamates **8** are obtained by treatment of amine **6** with the corresponding chloroformate. Amides **7** are obtained by reaction of **6** with the corresponding anhydride or hydrochloric acid, and thioureas **9** are obtained by reaction of **6** with ammonium thiocyanate. Finally, treatment of amides **7** with Lawesson reagent gives thioamides **10.**

Scheme 3 illustrates the process to get precursors with an alkoxymethylenic moiety in C5 by Williamson reaction of mesilates **5.**

According to Scheme 4, acylation of alcohol **4** with the corresponding anhydride or acid chloride allows the introduction of alkoxycarbonylic functionality in C5.

Finally, according to Scheme 5, introduction of secondary amine radicals (from weak nucleophilic amines) is carried out by activation of the amines through the corresponding *t*-Boc derivative, generation of the corresponding anion with a strong base (e.g. NaH) and nucleophilic attack of the anion over the mesilate **5**. Subsequently, deprotection of the corresponding *t*-Boc derivative **13** is carried out by treatment with an acid (e.g. CF₃-COOH) giving aminomethylen substituted compounds **14.**

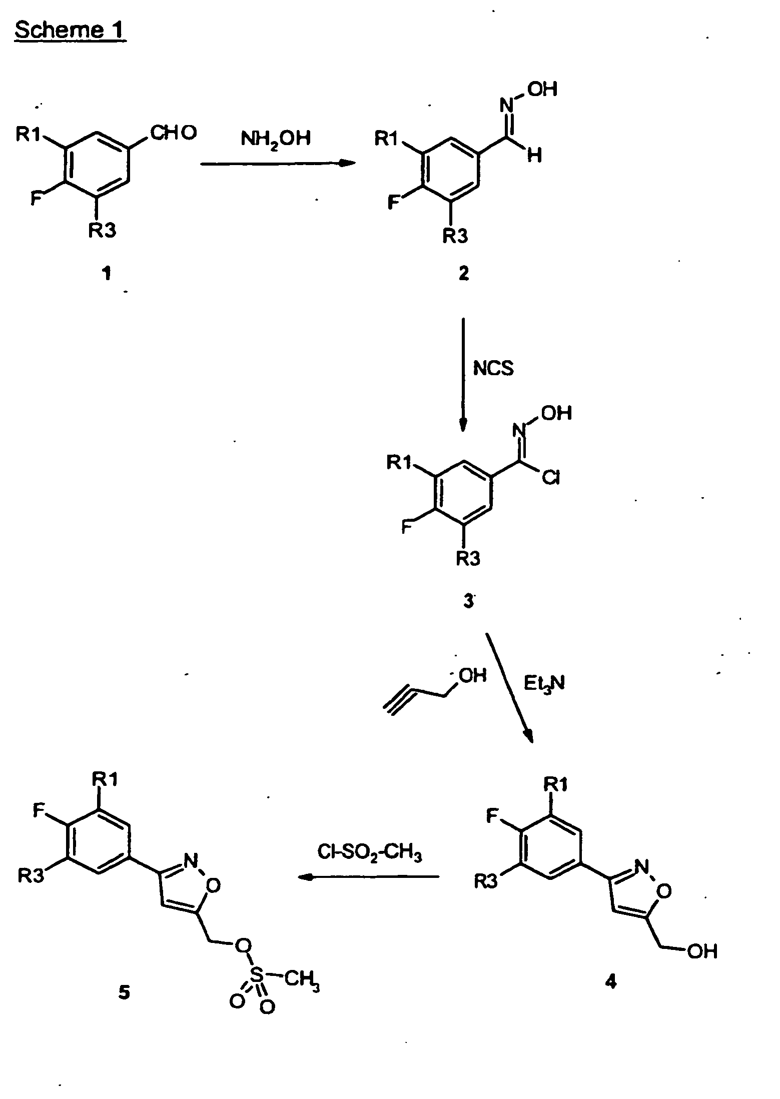

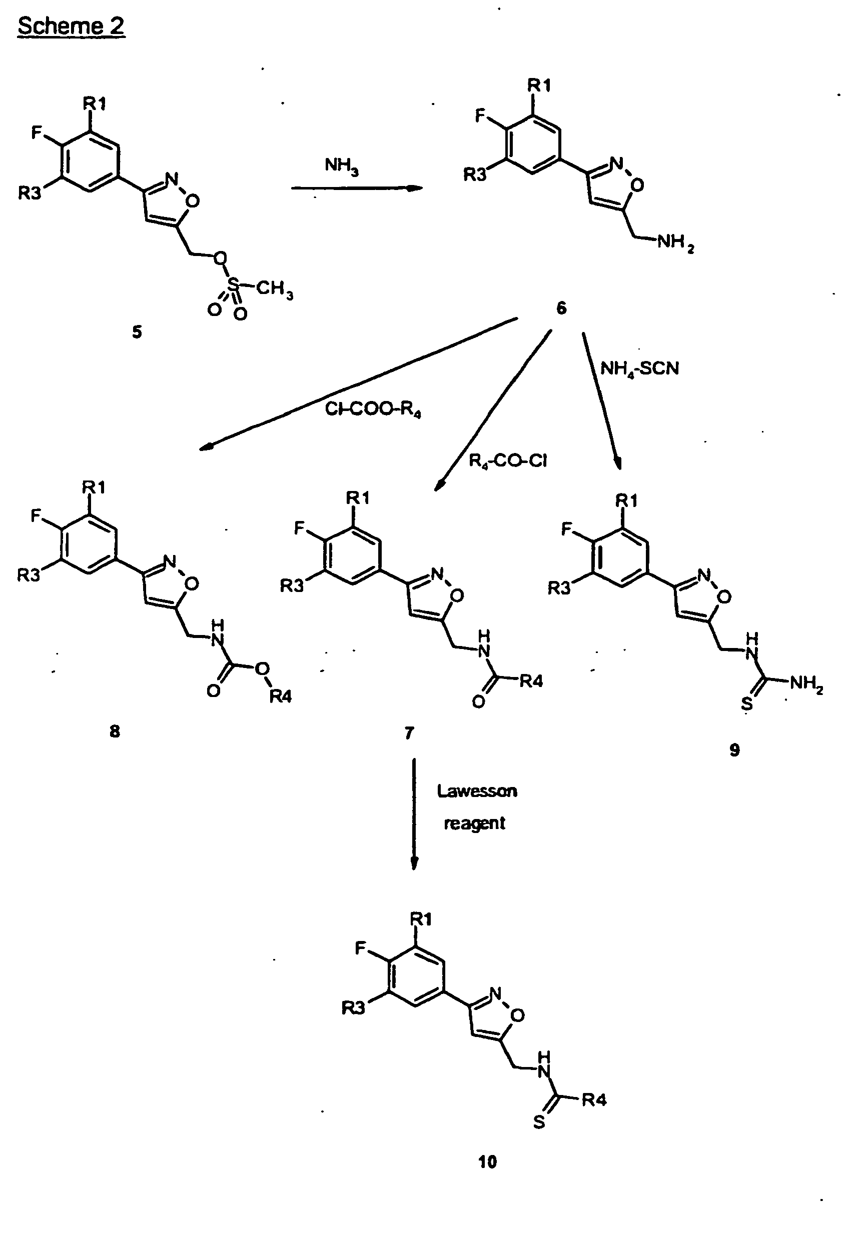

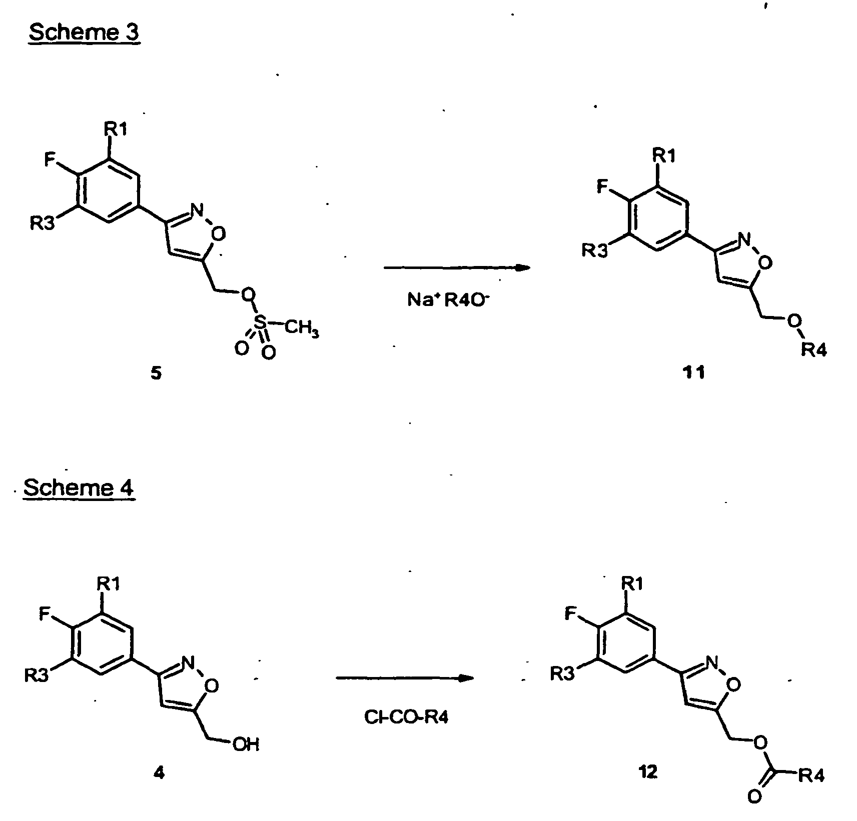

Subsequently, two general processes to obtain the final compounds of general formula (I) are disclosed.

According to Scheme 6, nucleophilic aromatic substitution through the attack of the fluorine derivative by the corresponding azolic type nucleophile, allows introduction of radicals of the characteristics mentioned in the last synthetic step giving azoles (Ia). In this reaction strong bases such as NaH, K₂CO₃ and potassic *tert*-butoxide in solvents such as DMF, DMSO or *N*-methylpyrrolidone are used, being the temperature range very broad.

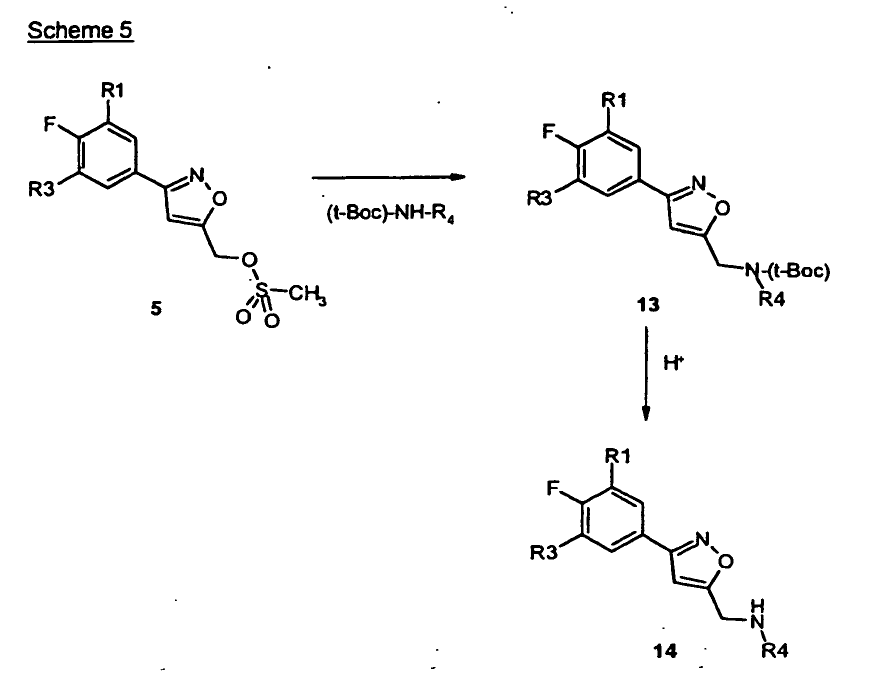

Introduction of alicyclic secondary amines such as morpholine, piperazine, or pyrrolidine is carried out in a similar way. In several cases, a great excess of nucleophilic agent is used, being the reaction carried out in a pressure reactor in such conditions that the amine is melt, and, in some cases, in the presence of an inorganic base such as anhydrous K₂CO₃.

When the amine is piperazine, functionalization of the NH free radical is produced by nucleophilic displacement of the corresponding R5 radical linked to a good leaving group ("Lg") as being attacked by the secondary piperazinic radical, to give final compounds (Ib).

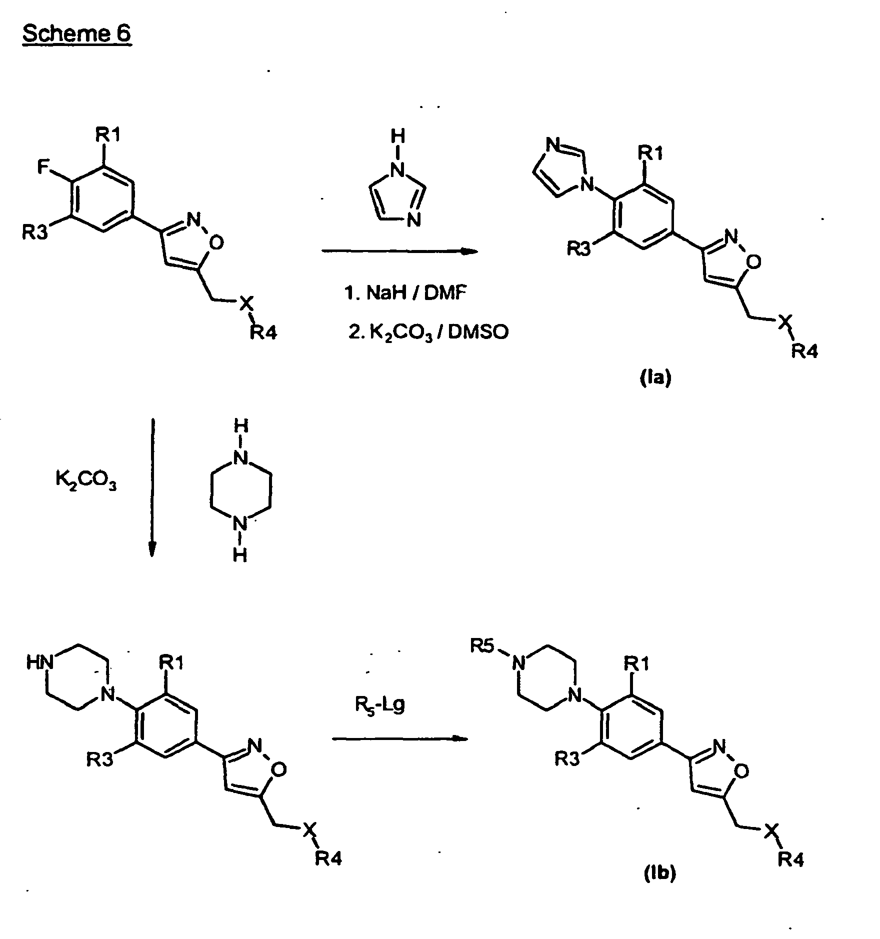

Nevertheless, when a C-C bond between the phenylic ring and the new bounded ring is sought, the process carried out substantially differs from those previously shown.

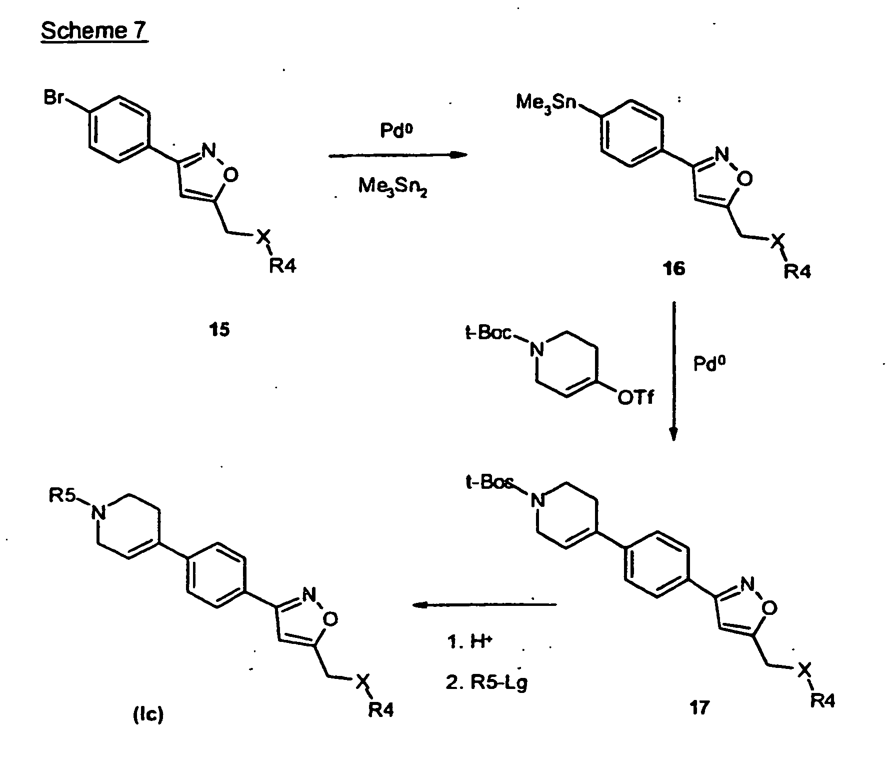

As shown in Scheme 7, in such cases the starting compound is the bromine derivative **15** obtained by one of the processes described above, to give the organometallic derivative **16**, which reacts with the corresponding triflate radical in the presence of metal palladium by a Stille reaction to give the (*t*-Boc) derivative **17.** Subsequent functionalization steps are equal to those described in some of the preceding processes.

Compounds of the present invention are useful in human and animal therapy, especially in the treatment of microbial infections and in the treatment of cancerous and precancerous pathologies. Preferably, they are administered by oral, parenteral or topical route. Therefore, according to other aspects of the invention there are provided the use of compounds of formula (I) for the preparation of a medicament for the treatment of the aforementioned pathologies, and the pharmaceutical compositions comprising at least a therapeutically effective quantity of the compound defined in any of claims 1 or 2, as the active principle, and pharmaceutically acceptable excipients or solvents.

The invention will be illustrated by the following non-limiting examples.

### Examples of antimicrobial activity

In order to assess the antimicrobial activity of the compounds of the present invention a method of microdilution in microtiter plate was used. The compounds were diluted in a nutritious medium and, subsequently, distributed by two-fold serial dilutions in 96 well plates. Then, plates were inoculated with a bacterial suspension. After incubation for 24 h at 35°C the minimum inhibitory concentration (MIC) of the drug in µg/mL was determined as the lowest concentration of compound which inhibits the growth of the bacterium. Results included in Table 1 illustrate the antimicrobial activity of some of the compounds of the present invention in comparison with thus obtained with two compounds (linezolid and eperezolid) of a known antimicrobial activity. The antimicrobial activity of the compound *versus Streptococcus faecalis* (BCM-010, strain designation as for SALVAT collection) *Staphylococcus aureus* (BCM-012, strain designation as for SALVAT collection) and *Moraxella catarrhalis* (BCM-015, strain designation as for SALVAT collection), respectively, is shown in the different columns.

**TABLE 1**

| COMPOUND | BCM- 010 MIC (µg/mL) | BCM- 012 MIC (µg/mL) | BCM-015 MIC (µg/mL) |
|---|---|---|---|
| 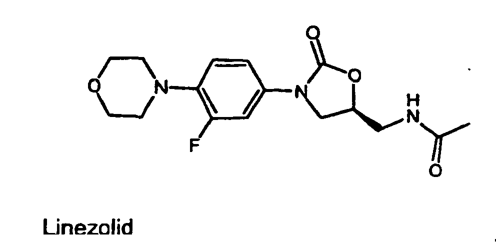 | 4 | 2 | 4 |
| 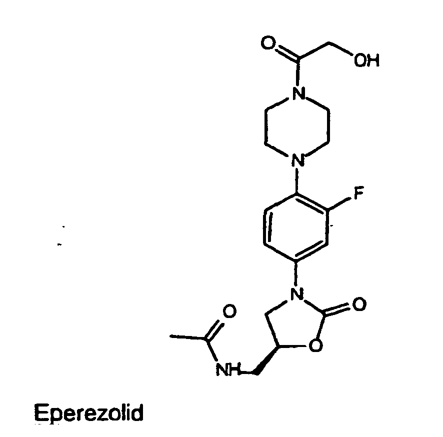 | 4 | 2 | 8 |
| 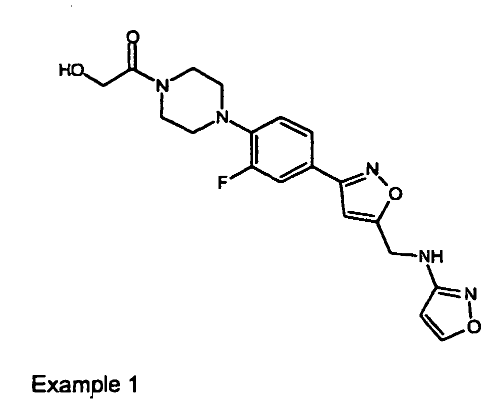 | 4 | 2 | 8 |
| 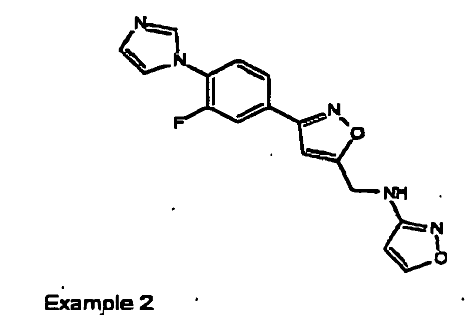 | 4 | 2 | |
| 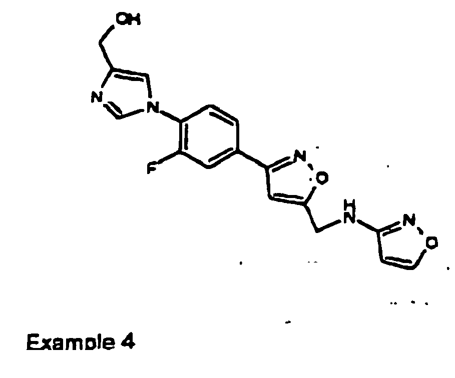 | 4 | 2 | |
| 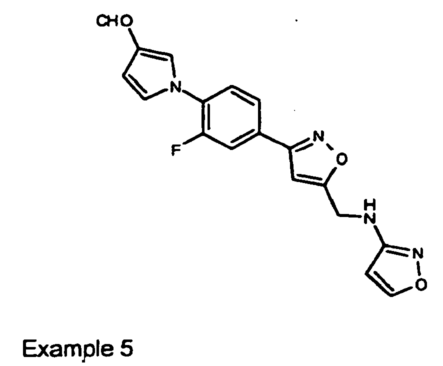 | 1 | 1 | 8 |
| 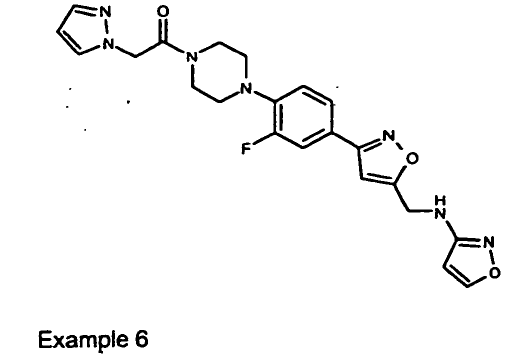 | 4 | 2 | >16 |
| 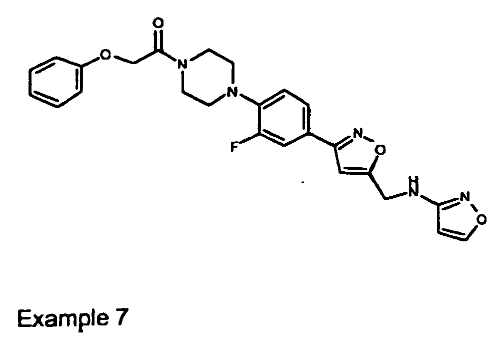 | 4 | 2 | |
| 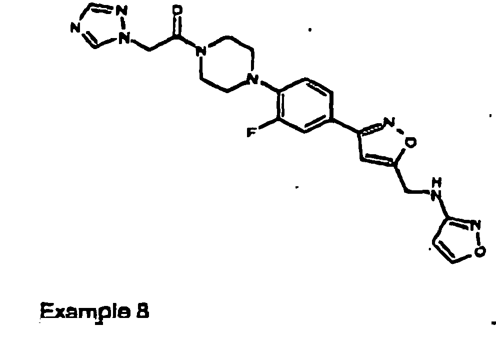 | 4 | 2 | |
| 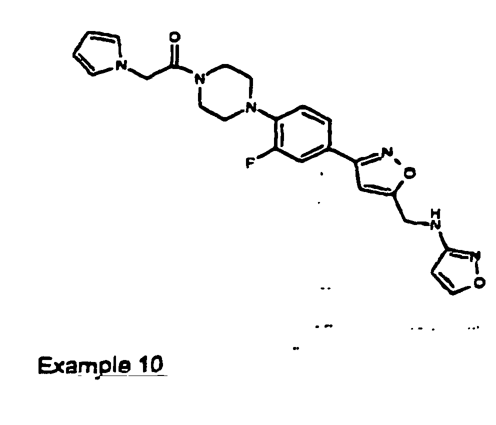 | 4 | 2 | >16 |
| 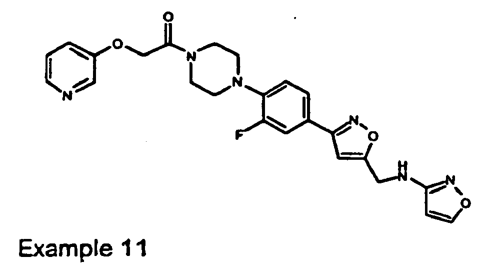 | 4 | 2 | |
| 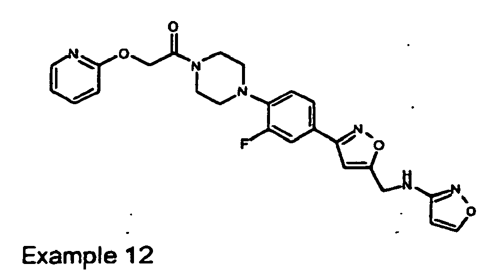 | 2 | 2 | |
| 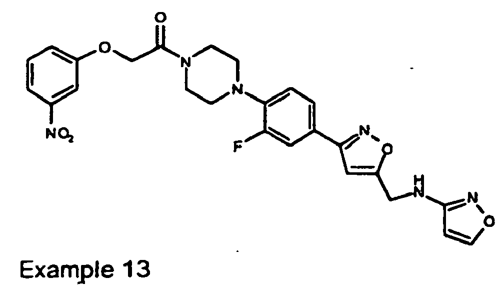 | 2 | 1 | |
| 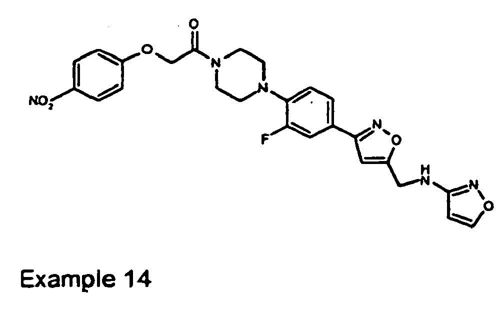 | 2 | 1 | |
| 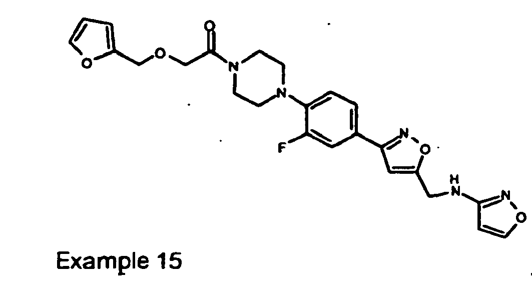 | 4 | 2 | |
| 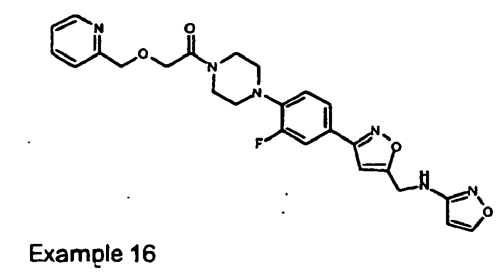 | 4 | 2 | |
| 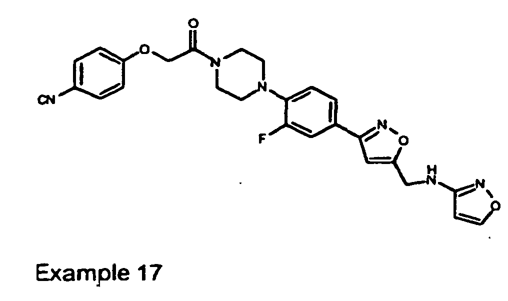 | 2 | 2 | |
| 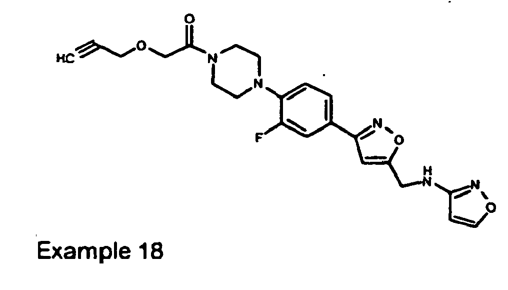 | 4 | 2 | |
| 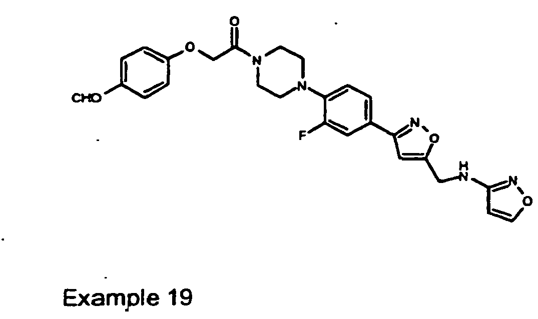 | 0.5 | 1 | |
| 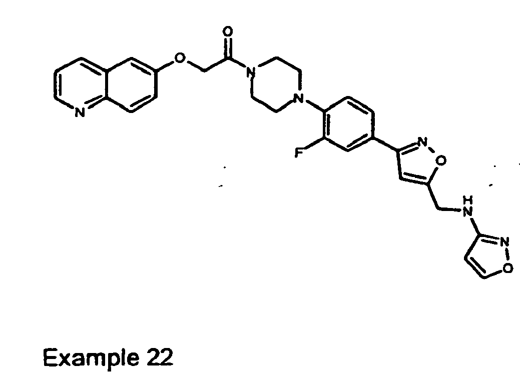 | 1 | 0.5 | 4 |
| 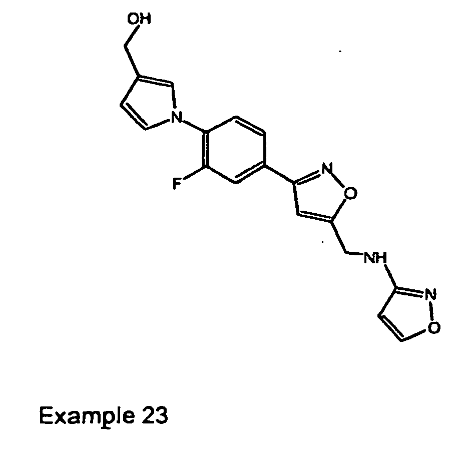 | 1 | 0.5 | 4 |
| 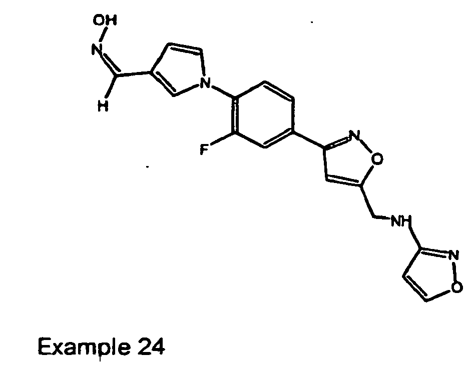 | 0.5 | 0.5 | 16 |
| 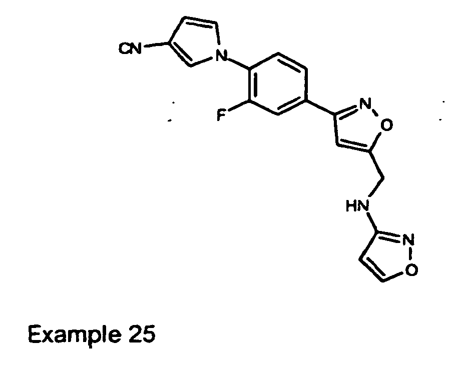 | 0.5 | 0.5 | 16 |
| 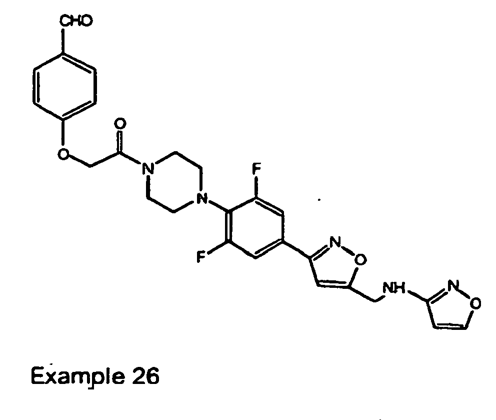 | 1 | 0.5 | 4 |
| 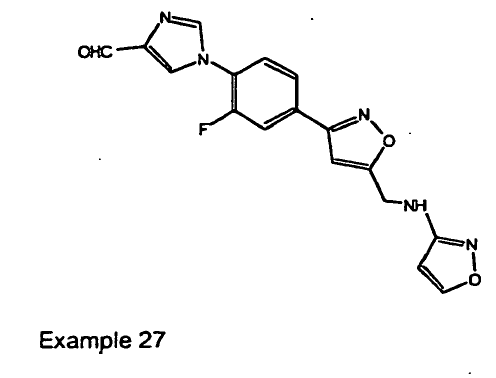 | 2 | 0.5 | 4 |
| 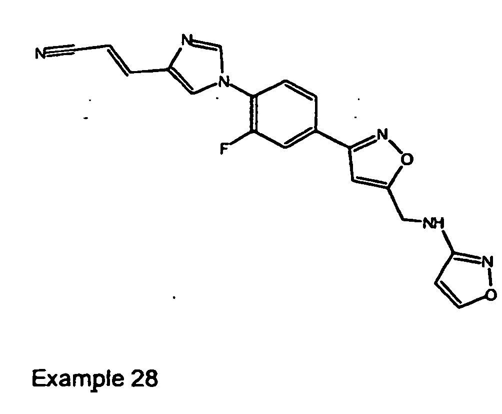 | 1 | 0.5 | 4 |
| 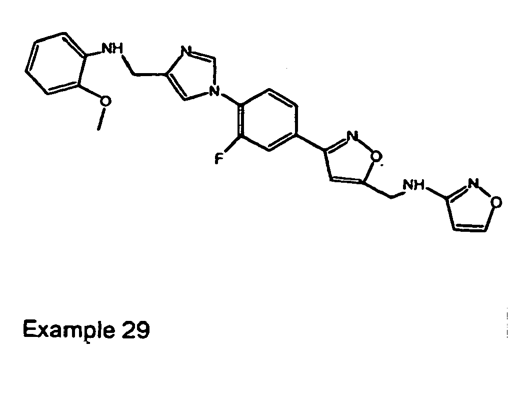 | 1 | 0.5 | 4 |
| 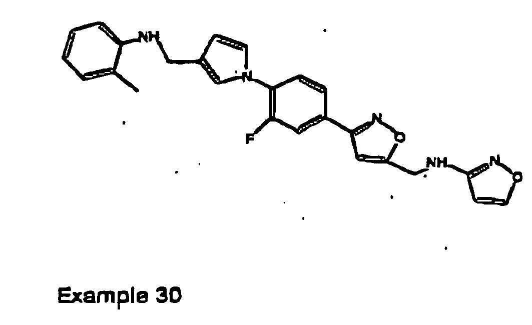 | 1 | 0.5 | 16 |
| 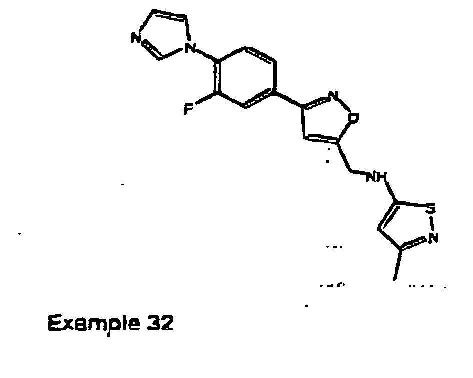 | 1 | 1 | - |
| 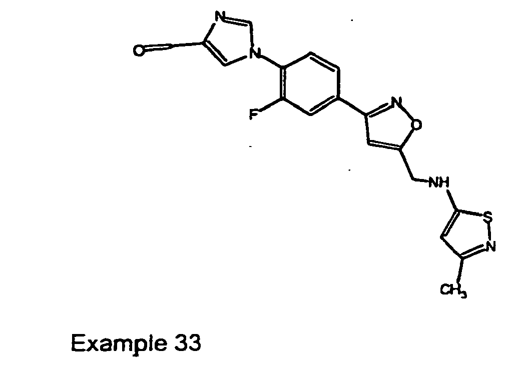 | 2 | 0.5 | 4 |
| 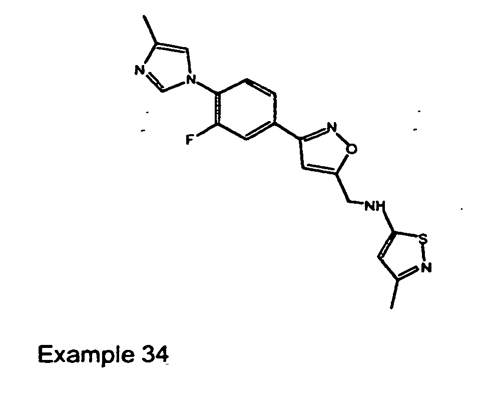 | 1 | 1 | 4 |

### Antitumoral activity examples

Antitumoral activity of the compounds of the present invention was evaluated by determining *in vitro* cell growth inhibition of 2 human colon adenocarcinome cell lines. The SBR (Sulphorhodamine B) protein dye assay described by the NCI (National Cancer Institute) was used. Results of Table 2 illustrate the antiproliferative activity of some of the compounds of the present invention versus the one obtained with exisulind (sulindac sulfone).

**TABLE 2**

| COMPOUND | Cell viability percentage at 100 µM | |
|---|---|---|
| | HT-29 | HCT-116 |
| 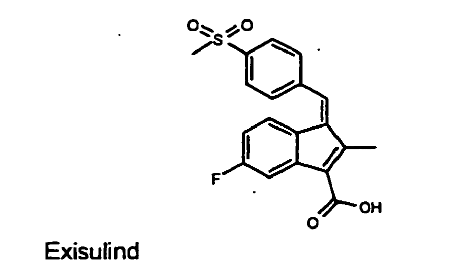 | 38.1 | 44.1 |
| 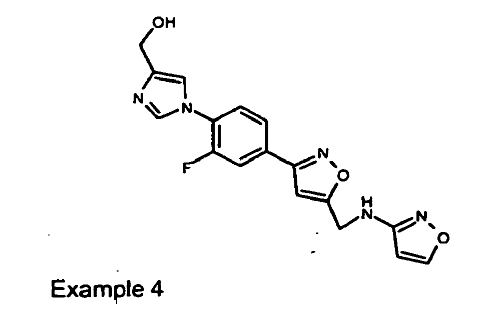 | 66.1 | 57.1 |
| 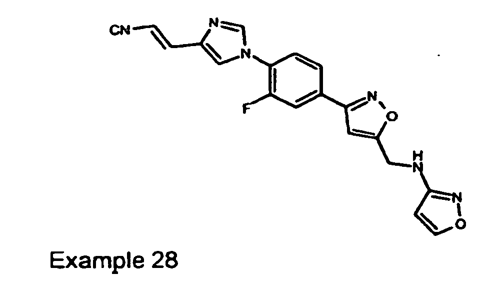 | 71.2 | 64.7 |
| 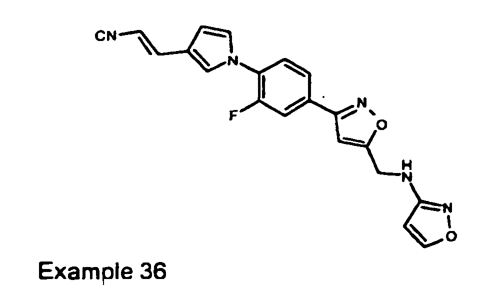 | 14.3 | 77.1 |
| 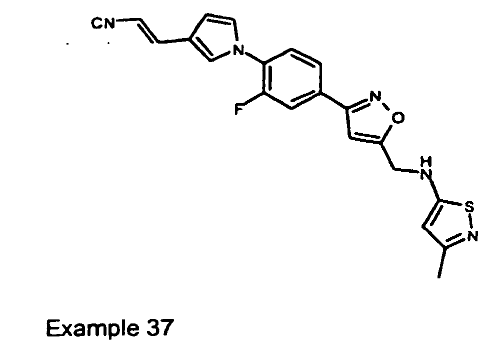 | <5 | 18.9 |
| 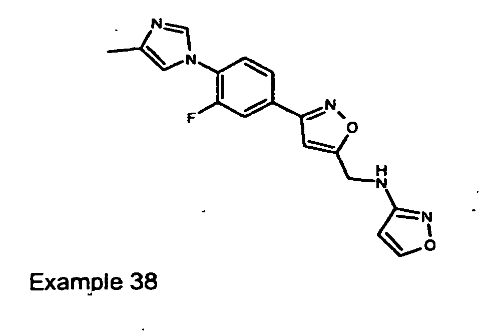 | 53.3 | 48.6 |

### Intermediate 1. Preparation of 3,4-difluorobenzaldoxime

A solution of 74.8 g (1.845 mol) of sodium hydroxide in 330 mL of deionized water was prepared and allowed to cool down. To the solution 150.0 g (1.049 mol) of 3,4-difluorobenzaldehyde were added and, then, 78.0 g (1.222 mol) of hydroxylamine hydrochloride were added dropwise, while an increase of the temperature over 22-25°C was avoided by refrigeration of the system with a water bath. The mixture was stirred mechanically at room temperature during 30 minutes, poured over 2.5 L of water, acidified with an aqueous solution of hydrochloric acid 6N to pH 6, and extracted with diethyl ether (3 x 1 L). The organic extracts were dried over anhydrous sodium sulfate and filtered, and the solvent was distilled off under reduced pressure. The residual solid was broken up with hexane to give 122.1 g (yield = 84%) of a yellow solid corresponding to the title compound. IR (KBr): 3327, 1694 cm⁻¹. Mass spectrum (m/e): 157 (M⁺).

### Intermediate 2. Preparation of 3,4-difluoro-N-hydroxybenzenecarboxiimidoyl chloride

A solution of 17.0 g (0.108 mol) of 3,4-difluorobenzaldoxime (Intermediate 1) in 1.7 L of *N*,*N*-dimethylformamide was prepared and externally cooled down with an ice bath to an internal temperature comprised between 0 and 5°C. Then, 26.0 g (0.195 mol) of *N*-chlorosuccinimide were added under nitrogen atmosphere. The mixture was heated at 50°C during 3 h, allowed to cool down at room temperature, and then poured over 1.7 kg of ice while keeping mechanical stirring during 30 minutes. The mixture was extracted three times with 1 L of toluene. The organic extracts were washed with 1 L of water, and then three times with 1 L of a saturated sodium chloride aqueous solution. The organic layer was dried over anhydrous sodium sulfate and filtered. The solvent was distilled off under reduced pressure. The residual solid was broken up with hexane to give 17.6 g (yield = 85%) of a yellow solid corresponding to the title compound. IR (KBr): 3435, 1618 cm⁻¹. Mass spectrum (m/e): 192 (M⁺).

### Intermediate 3. Preparation of [3-(3,4-difluoropheny)isoxazol-5-yl]methanol

A solution of 71 g (0.37 mol) of 3,4-difluoro-*N*-hydroxybenzenocarboximidoyl chloride (Intermediate 2) in 1 L of toluene was prepared and cooled down with an ice bath. After adding 45 mL of triethylamine dropwise and with stirring, a solution of 41.5 g (0.74 mol) of propargylic alcohol in 200 mL of toluene was added. The mixture was stirred at room temperature for 48 h. Then, 650 mL of water were added and the organic layer was separated, dried over anhydrous sodium sulfate and evaporated to dryness. The resulting solid was broken up with hexane. 56 g (yield = 71%) of a beige solid were obtained. IR (KBr): 3380, 1612, 1600, 1500 cm⁻¹. Mass spectrum (m/e): 211 (M⁺).

### Intermediate 4. Preparation of 3-(3,4-difluorophenyl)isoxazol-5-methyl methylsulfonate

A solution of 32 g (0.151 mol) of [3-(3,4difluorophenyl)isoxazol-5-yl]methanol (Intermediate 3) in 1200 mL of dichloromethane was prepared and cooled down with an ice bath to a temperature comprised between 0 and 5°C. 35 mL of triethylamine were added, and then a solution of 17.5 mL (25.9 g, 0.226 mol) of methanesulfonyl chloride in 20 mL of dichloromethane was added dropwise. The reaction mixture was stirred and allowed to warm to room temperature for 2 h. The crude product was poured over 1.5 L of water/ice, and the mixture was placed in a separatory funnel. The organic layer was separated, washed three times with 1 L of a 5% sodium bicarbonate aqueous solution, dried over anhydrous sodium sulfate, and filtered. The solvent was distilled off under reduced pressure and the residual solid broken up with hexane. 42 g (yield = 96%) of a beige solid were obtained. IR (KBr): 1620, 1600, 1500, 1320, 1180 cm⁻¹. Mass spectrum (m/e): 289 (M⁺).

### Intermediate 5. Preparation of isoxazol-3-ylcarbamic acid, tert-butyl ester

A mixture of 28.5 g (0.338 mol) of 3-aminoisoxazole in 950 mL of dichloromethane was prepared, and 2.8 g (0.023 mol) of 4-dimethylaminopyridine, and 147.7 g (0.677 mol) of di-*tert*-butyl dicarbonate were added. The mixture was stirred at room temperature for 18 h, and the solvent was distilled off under reduced pressure. The residue was dissolved in 570 mL of methanol, and 180 mL of a 2 N sodium hydroxide aqueous solution were added. The mixture was stirred for 2 h, and then 450 mL of an 10% citric acid aqueous solution were added to adjust pH between 4 and 5. Stirring was continued for some minutes and the solution was poured over 2.8 L of water. The solid was filtered and dissolved in 500 mL of dichloromethane. The solution was dried over anhydrous sodium sulfate, and filtered, and the solvent was distilled off under reduced pressure. The residual solid was broken up with hexane. 42. 9 g (yield = 69%) of a yellow solid were obtained. IR (KBr): 3257, 1732 cm⁻¹. Mass spectrum (m/e): 184 (M⁺).

### Intermediate 6. Preparation of isoxazol-3-yl[3-(3,4-difluorophenyl)isoxazol-5-ylmethyl]carbamic acid, tert-butyl ester

A solution of 34.4 g (0.186 mol) of isoxazol-3-ylcarbamic acid, *tert*-butyl ester (Intermediate 5) in 750 mL of *N*,*N*-dimethylformamide was prepared and cooled down with an ice bath to a temperature comprised between 0 and 5°C. 7.5 g (0.187 mol) of a 60% sodium hydride suspension in paraffin were added under inert atmosphere, and the mixture was stirred at low temperature for 45 minutes. Then, 34.1 g (0.12 mol) of 3-(3,4-difluoro-phenyl)isoxazo1,5-methyl methylsulfonate (Intermediate 4) were added. The mixture was stirred at 40°C for 16 h, and then allowed to cool down at room temperature. The reaction crude was poured over 5 L of a 5% sodium bicarbonate aqueous solution and the final solution was extracted three times with 1.5 L of ethyl acetate. The organic layer was washed five times with 1 L of saturated sodium chloride aqueous solution, dried over anhydrous sodium sulfate, and filtered, and the solvent was distilled off under reduced pressure. The residual solid was broken up with hexane. 39 g (yield = 88%) of a yellow solid was obtained. IR (KBr): 1717 cm⁻¹. Mass spectrum (m/e): 391 (M⁺).

### Intermediate 7. Preparation of isoxazol-3-yl[3-(3,4-difluorophenyl)isoxazol-5-ylmethyl]amine

To 300 mL of a 10% w/v solution of concentrated sulfuric acid in dioxane, 10 g (0.0255 mol) of isoxazol-3-yl[3-(3,4-difluorophenyl)isoxazol-5-ylmethyl]-carbamic acid *tert*-butyl ester were added. The mixture was stirred at 30°C for 1 h and, then, allowed to cool down at room temperature. The solvent was distilled off under reduced pressure, and the residue was dissolved in 200 mL of water. The resulting solution was basified with concentrated ammonia, and extracted three times with 200 mL of dichloromethane. The extracts were washed twice with 200 mL of saturated sodium chloride aqueous solution, dried over anhydrous sodium sulfate, and filtered. The solvent was distilled off under reduced pressure, and the residual solid obtained was broken up with hexane. 4.95 g (yield = 79%) of a white solid were obtained. IR (KBr): 3380, 1620, 1600, 1500 cm⁻¹. Mass spectrum (m/e): 277 (M⁺).

### Intermediate 8. Preparation of (3-(3,4-difluorophenyl)isoxazol-yl]-methylamine

A solution of 17.5 g (0.060 mol) of 3-(3,4-difluorophenyl)isoxazol-5-methyl methylsulfonate (Intermediate 4) in 200 mL of methanol, and 100 mL of concentrated ammonia was prepared. After stirring for 48 h, the solvent was distilled off under reduced pressure. The residue was treated with 300 mL of ethyl acetate, and 100 mL of a saturated sodium chloride aqueous solution. The organic layer was washed twice with 100 mL of saturated sodium chloride aqueous solution, dried over anhydrous sodium sulfate and filtered. The solvent was distilled off under reduced pressure, and the residual solid obtained was broken up with hexane. 9.4 g (yield = 74%) of a yellow solid were obtained. IR (KBr): 3400, 3100, 1620, 1600, 1480 cm⁻¹. Mass spectrum (m/e): 210(M⁺).

### Intermediate 9. Preparation of N-[3-(3,4-difluorophenyl)isoxazol-5-ylmethyl]-acetamide

A solution of 1.2 g (5.68 mmol) of [3-(3,4-difluorophenyl)isoxazol-5-yl]-methylamine (Intermediate 8) in 100 mL of dichloromethane, and 10 mL of triethylamine was prepared. After cooling down the solution at a temperature comprised between 0 and 5°C with an ice bath, a solution of 0.5 g (6.36 mmol) of acetyl chloride in 5 mL of dichloromethane was added dropwise. The mixture was stirred at room temperature for 1 h. Then, 100 mL of dichloromethane were added and the solution was washed three times with 100 mL of a 5% sodium bicarbonate aqueous solution, then with water, and eventually was dried over anhydrous sodium sulfate and filtered. The solvent was distilled off under reduced pressure. The residual solid obtained was broken up with diethyl ether. 900 mg (yield = 62%) of a white solid were obtained. IR (KBr): 3180, 1660 cm⁻¹. Mass spectrum (m/e): 253 (M⁺).

### Intermediate 10. Preparation of [3-(3-fluoro-4-piperazin-1-ylphenyl)isoxazol-5-ylmethy]isoxazol-3-ylamine

In a pressure reactor, a mixture, previously ground in a mortar, of 8.2 g (0.03 mol) of isoxazol-3-yl[3-(3,4-difluoro-phenyl)isoxazot-5-ylmethyl]-amine (Intermediate 7), 44 g de piperazine, and 6.8 g of anhydrous potassium carbonate were placed. The mixture was heated at a temperature comprised between 130 and 135 °C for 6 h, and then allowed to cool down. The reaction crude was treated with 500 mL of chloroform, and 500 mL of water. The organic layer was washed three times with 100 mL of water, dried over anhydrous sodium sulfate, and filtered. The solvent was distilled off under reduced pressure. The residue was filtered through a short pad of silica gel with elution by a mixture of dichloromethane:ethyl acetate (5:1). 4.2 g (yield = 42 %) of a white solid. IR(KBr): 3206, 1613 cm⁻¹. Mass spectrum (m/e): 343 (M⁺). ¹H-NMR (200 MHz, d₆-DMSO, δ ppm): 8.42 (s, 1H), 7.70-7.60 (m, 2H), 7.25-6.90 (m, 3H), 6.00 (s, 1H), 4.45 (s, 2H), 3.40-3.25 (m, 8H).

### Intermediate 11. Preparation of 2-chloro-1-(4-{2-fluoro-4-[5-(isoxazol-3-ylaminomethyl)isoxazol-3-yl]phenyl}piperazin-1-yl)ethanone

A solution of 1.75 g (5.09 mmol) of [3-(3-fluoro-4-piperazin-1-ylphenyl)isoxazol-5-ylmethyl]isoxazol-3-ylamine (Intermediate 10) in 40 mL of dichloromethane was prepared and cooled down to a temperature comprised between 0 and 5°C with an ice bath. After adding 1.2 mL of triethylamine, a solution of 1 g (8.84 mmol) of acetyl chloride in 5 mL of dichloromethane was added dropwise. The mixture was stirred at room temperature for 1h and diluted with 40 mL of dichloromethane. The solution was washed three times with 50 mL of a 5% sodium bicarbonate aqueous solution, dried over anhydrous sodium sulfate, filtered, and evaporated to dryness. The residue was filtered over silica gel with a mixture of dichloromethane/ethyl acetate (1:1) as eluanteluant. 1.1 g (yield = 51%) of a slightly yellow solid was obtained. IR (KBr): 3300, 1650 cm⁻¹. Mass spectrum (m/e): 420 (M⁺).

### Intermediate 12. Preparation of 3,4,5-trifluorobenzaldoxime

Following an analogous process to that described in preparation of Intermediate 1, 1.54 g (yield = 80%) of a yellow solid corresponding to the title compound were obtained. IR (KBr): 3324, 1705 cm⁻¹. Mass spectrum (m/e): 175 (M⁺).

### Intermediate 13. Preparation of 3,4,5-difluoro-N-hydroxybenzenecarboxiimidoyl chloride

Following an analogous process to that described in preparation of Intermediate 2, 19 g (yield = 64%) of a yellow oil corresponding to the title compound were obtained. IR (KBr): 3350, 1707 cm⁻¹.

### Intermediate 14. Preparation of [3-(3,4,5-trifluorophenyl)isoxazol-5-yl]methanol

Following an analogous process to that described in preparation of Intermediate 3, 18 g (yield = 83%) of a brown solid were obtained. Mass spectrum (m/e): 229 (M⁺).

### Intermediate 15. Preparation of 3-(3,4,5-trifluorophenyl)isoxazol-5-methyl methylsulfonate

Following an analogous process to that described in preparation of Intermediate 4, 28 g (yield = 66%) of a beige solid were obtained. IR (KBr): 1351, 1183 cm⁻¹.

### Intermediate 16. Preparation of isoxazol-3-yl[3-(3,4,5-trifluorophenyl)isoxazol-5-ylmethyl]carbamic acid, tert-butyl ester

Following an analogous process to that described in preparation of Intermediate 6, 30 g (yield = 93%) of a brown oil were obtained. IR (KBr): 1730 cm⁻¹.

### Intermediate 17. Preparation of isoxazol-3-yl[3-(3,4,5-trifluorophenyl)isoxazol-5-ylmethyl]amine

Following an analogous process to that described in preparation of Intermediate 7, 1.8 g (yield = 60%) of an orange solid were obtained. IR (KBr): 3264.

### Intermediate 18. Preparation of [3-(3,5-difluoro-4-piperazin-1-ylphenyl)-isoxazol-5-ylmethyl]isoxazol-3-ylamine

Following an analogous process to that described in preparation of Intermediate 10, 1.3 g (yield = 23%) of a white solid were obtained. IR(KBr): 3386,3287,1597 cm⁻¹. ¹H-NMR (200 MHz, d₆-DMSO, δ ppm): 8.11 (d, 1H), 7.41 (s, 1H), 7.38 (s, 1H), 6.57 (s, 1H), 6.42 (t, 1H), 5.89 (d, 1H), 4.46 (d, 2H), 3.40-2.9 (m, 8H).

### Intermediate 19. Preparation of 3-methylisothiazol-5-ylcarbamic acid, tert-butyl ester

A mixture of 5.0 g (33.2 mmol) of 5-amino-3-methylisothiazole hydrochloride, and 4.3 g (33.3 mmol) of *N,N*-diisopropylethylamine in 100 mL of dichloromethane was prepared and stirred at room temperature for 30 minutes. 0.30 g (2.43 mmol) of 4-methylaminopyridine, and 15.69 g (71.9 mmol) of di-*tert*-butyl dicarbonate were added. The mixture was stirred at room temperature for 18 h, and the solvent was distilled off under reduced pressure. The residue was dissolved in 62 mL of methanol and 20 mL of a 2 N sodium hydroxide solution was added. The mixture was stirred during 2 h. 50 mL of a 10% citric acid solution was added to adjust pH between 4 and 5, and the mixture was stirred again for some minutes, poured over 130 mL of water, and filtered. The solid obtained was dissolved in 50 mL of dichloromethane. The solution was dried over anhydrous sodium sulfate and filtered, and the solvent was distilled off under reduced pressure to give a crude oil which crystallizes slowly. 2.57 g (yield = 36%) of a yellow solid were obtained. Mass spectrum (m/e): 215 (M⁺).

### Intermediate 20. Preparation of [3-(3,4-difluorophenyl)isoxazol-5-ylmethyl]-(3-methylisotiazol-5yl)carbamic acid, tert-butyl ester

Following an analogous process to that described in preparation of Intermediate 6, 2.21 g (yield = 92%) of an orange solid corresponding to the title compound were obtained. IR (KBr): 1695 cm⁻¹.

### Intermediate 21. Preparation of [3-(3,4-difluorophenyl)isoxazol-5-ylmethyl]-(3-methylisotiazol-5-yl)amine

Following an analogous process to that described in preparation of Intermediate 7, 1.06 g (yield = 63%) of an orange solid corresponding to the title compound were obtained. IR (KBr): 3374 cm⁻¹.

### Intermediate 22. Preparation of isoxazol-3-ylmethanol

To a solution of 30 g (0.306 mol) of ethyl propiolate in 600 mL of ethanol, 85 g (1.223 mol) of hydroxylamine hydrochloride in 1.1 L of a 10% sodium hydroxide solution were added in an argon atmosphere. The mixture was stirred at room temperature for 48 h, and then acidified to a pH = 2 -3 by addition of concentrated hydrochloric acid. The reaction mixture was extracted three times with diethyl ether, and the combined extracts were washed with saturated sodium chloride solution, dried over anhydrous sodium sulfate, and filtered. The solvent was distilled off under reduced pressure. The residue was extracted with hot hexane, and then the solvent was evaporated under reduced pressure. 9.0 g (yield = 35%) of a white crystalline solid corresponding to the title compound was obtained. Mass spectrum (m/e): 83 (M⁺).

### Intermediate 23. Preparation of 3-(3,4-difluorophenyl)-5-(isoxazol-3-yloximethyl)isoxazole

In 55 mL of *N,N*-dimethylformamide a suspension of 2.12 g (53 mmol) of 60% sodium hydride in paraffin was prepared under argon atmosphere. After cooling to 0°C, a solution of 4.5 g (53 mmol) of isoxazol-3-ylmethanol (Intermediate 22) in 50 mL of *N*,*N*-dimethylformamide was added dropwise. The mixture was stirred at 40°C for 15 minutes, and then allowed to cool down at room temperature. A solution of 15.2 g (52.4 mmol) of 3-(3,4-difluorophenyl)isoxazol-5-methyl methylsulfonate (Intermediate 4) in 110 mL de *N,N*-dimethylformamide was added dropwise. The mixture was stirred at 70°C for 1 h, cooled, poured over 1.7 L of a 5% sodium bicarbonate solution, and extracted three times with ethyl acetate. The combined organic extracts were washed with 1.7 L of deionized water, and with 1.7 L of a saturated solution of sodium chloride, then dried over anhydrous sodium sulfate, and filtered. The solvent was distilled off under reduced pressure. The residue was chromatographed on a silica gel column, eluting with dichloromethane. Relevant fractions were combined to give, once evaporated the solvent, 14.5 g (quantitative yield) of a white solid. Mass spectrum (m/e): 278 (M⁺).

### Example 1. Preparation of 3-[3-fluoro-4-(hydroxyacetyl)piperazin-1-ylpheny]-5-(isoxazol-3-ylaminomethyl)isoxazole

To a solution of 2.0 g (5.8 mmol) of 3-(3-fluoro-4-piperazin-1-ylphenyl)-5-(isoxazol-3-ylaminomethyl)isoxazole (Intermediate 10) in 40 mL of dichloromethane were added 1.37 mL (1.0 g, 9.9 mmol) of triethylamine at 0°C. Then, 1.1 mL (1.3 g, 9.9 mmol) of acetoxyacetyl chloride were added dropwise. The mixture was stirred for 1 h while allowing to cool down at room temperature. The crude product was diluted with 600 mL of dichloromethane, and washed with a 5% sodium bicarbonate solution. The organic layer was dried over anhydrous sodium sulfate, and filtered, and the solvent was distilled off by reduced pressure. The resulting solid was broken up with hexane. 2.22 g (yield = 88%) of 3-[3-fluoro-4-(acetoxyacetyl)piperazin-1-ylphenyl]-5-(isoxazol-3-ylaminomethyl)isoxazol were obtained. IR (KBr): 3300, 1747, 1650 cm⁻¹.¹H-NMR (200 MHz, CDCl₃/d₆-DMSO, δ ppm): 8.39 (d, 1H), 7.65 (m, 1H), 7.59 (s, 1H), 7.15 (m, 1H), 6.90 (m, 2H), 6.04 (d, 1H), 4.81 (s, 2H), 4.45 (d, 2H), 3.56 (m, 4H), 3.08 (m, 4H), 2.08 (s, 3H).

A mixture of 2.0 g (4.5 mmol) of the previous product and 1.24 g (9 mmol) of potassium carbonate in 190 mL of methanol was stirred at room temperature for 30 minutes. The carbonate was filtered and the solvent distilled off by reduced pressure. The crude product was treated with water, and the solid was filtered and washed with isopropyl alcohol and diethyl ether. 1.31 g (yield = 73%) of a beige solid corresponding to the title compound were obtained. IR (KBr): 3500, 3315, 1635 cm⁻¹. ¹H-NMR (200 MHz, CDCl₃/d₆-DMSO, δ ppm): 8.44 (s, 1H), 7.64 (m, 1H), 7.60 (s, 1H), 7.11 (m, 1H). 6.89 (m, 2H), 6.03 (d, 1H), 4.65 (br, 1H), 4.44 (d, 2H), 4.15 (s, 2H), 3.62 (m, 4H), 3.50 (m, 4H).

### Example 2. Preparation of 3-(3-fluoro-4-imidazol-1-ylphenyl)-5-(isoxazol-3-ylaminomethyl)isoxazole

A mixture of 300 mg (4.36 mmol) of imidazol, 40 mL of dimethylsulfoxide, 1.20 g (8.72 mmol) of anhydrous potassium carbonate and 1 g (3.61 mmol) of 3-(3,4-difluorophenyl)-5-(3-isoxazoylaminomethyl)isoxazole (Intermediate 7) was heated at 90 °C for 24 h with stirring under an inert atmosphere. The reaction mixture was allowed to cool down, poured over 400 mL of a saturated sodium chloride solution, and extracted three times with 200 mL of ethyl acetate. The organic extracts were combined, dried over anhydrous sodium sulfate, filtered, and evaporated to dryness. The residue was chromatographed on a silica gel column with dichloromethane/methanol (20:1) as eluant. Relevant fractions were combined to give, once evaporated the solvent, 380 mg (yield = 32%) of a slightly yellow solid. IR: 3300, 1595 cm⁻¹. Mass spectrum (m/e): 328 (M⁺). ¹H-NMR (200 MHz, CDCl₃, δ ppm): 8.4 (s, 1 H), 8.05 (s1 H). 8.00-6.90 (m, 6H), 8.0 (s, 1H), 4.40 (d, 2H).

### Example 4: Preparation of 3-[3-fluoro-(4-hydroxymethylimidazol-1-yl)pheny)]-5-(isoxazol-3-ylaminomethyl)isoxazole

A mixture of 600 mg (4.36 mmol) of 4-hydroxymethylimidazol hydrochloride, 1.84 g (13.4 mmol) of anhydrous potassium carbonate, 40 mL of dimelhylsulfoxide, and 1 g (3:61 mmol) of 3-(3,4-diftuorophenyl)-5-(3-isoxazoylaminomethyl)isoxazole (Intermediate 7) was heated at 90°C with stirring for 24 h under an inert atmosphere, allowed to cool, poured over . 400 mL of a saturated sodium chloride solution, and extracted three times with 300 mL of ethyl acetate. The extracts were dried over anhydrous sodium sulfate, and filtered. The solvent was distilled off under reduced pressure and the obtained residue was chromatographed on a silica gel column with dichloromethane/methanol (20:1) as eluant. 300 mg (yield = 23 %) of a yellow solid were obtained. IR (KBr): 3300, 3100, 1595 cm⁻¹. Mass spectrum (m/e): 355 (M⁺). ¹H-NMR (200 MHz, CDCl₃, δ ppm): 8.44 (s, 1 H), 8.20-6.95 (m, 8H), 6.1 (s, 1H), 4.50 (d, 2H), 3.30 (s, 2H), 3.00 (br, 2H).

### Example 5: Preparation of (1-{2-fluoro-4-[5-(isoxazol-3-ylaminomethyl)isoxazol-3-yl]phenyl}-1H-pyrrol-3-carboxaldehyde

A solution of 2.4 g (25.2 mmol) of 3-formylpyrrole in 90 mL of *N,N*-dimethylformamide was prepared under an inert atmosphere. After cooling with an ice bath to a temperature comprised between 0 and 5°C, 1 g (25 mmol) of 60% sodium hydride was added. The mixture was stirred for 1 h, allowing the temperature to rise to ambient. Then, 4.2 g (15 mmol) of 3-(3,4-difluorophenyl)-5-(isoxazol-3-ylaminomethyl)isoxazole (Intermediate 7) were added. The mixture was heated at 60°C with continued stirring for 6 h, then allowed to cool down, diluted with 1 L of ethyl acetate, and washed five times with 300 mL of saturated sodium chloride solution. The solution was dried over anhydrous sodium sulfate, and filtered, and the solvent was distilled off under reduced pressure. The obtained residue was chromatographed on a silica gel column with dichloromethane/ethyl acetate (5:1) as eluant. Relevant fractions were combined, and the solvent evaporated to give 1.7 g (yield = 32%) of a slightly yellow solid. Mass spectrum (m/e): 352 (M⁺). ¹H-NMR (200 MHz, CDCl₃, δ ppm): 8.44 (s, 1H), 8.20-6.95 (m, 8H), 6.1 (s, 1H), 4.50 (d, 2H).

### Example 6. Preparation of 3-[3-fluoro-4-(4-(1-pyrazolyl)acetyl)piperazin-1-yl)-phenyl]5-(isoxazol-3-ylaminomethyl)isoxazole

To a mixture of 200 mg (2.94 mmol) of pyrazole, 811 mg (5.87 mmol) of anhydrous potassium carbonate, and 40 mL of dimethylsulfoxide, 600 mg (1.42 mmol) of 2-chloro-1-(4-{2-fluoro-4-[5-(isoxazol-3-ylaminomethyl)isoxazol-3-yl]phenyl}piperazin-1-yl)ethanone (Intermediate 11) were added. After stirring at room temperature for 48h under an inert atmosphere, the mixture was diluted with 500 mL of ethyl acetate, washed four times with 200 mL of saturated sodium chloride solution, dried over anhydrous sodium sulfate, and filtered. The solvent was distilled off under reduced pressure and the obtained residue was chromatographed on a silica gel column with dichloromethane/methanol (20:1) as eluant. Relevant fractions were combined and the solvent evaporated. The residual solid was broken up with diethyl ether and filtered to give 580 mg (yield = 90%) of a white solid. IR (KBr): 3189, 1656 cm⁻¹. Mass spectrum (m/e): 451 (M⁺). ¹H-NMR (200 MHz, d₆-DMSO, δ(ppm)): 8.43 (d, 1H), 7.69-7.62 (m, 3H), 7.44 (d, 1H), 7.14 (t, 1H), 6.89 (s, 1H), 6.27 (t, 1H), 6.06 (d, 1H), 5.19 (s, 2H), 4.46 (s, 2H), 3.66 (m, 4H), 3.11 (m, 4H).

### Example 7. Preparation of 1-(4-{2-fluoro-4-[5-(isoxazol-3-ylaminomethyl) isoxazo-3-yl]-phenyl}piperazin-1-yl)-2-phenoxyethanone

To a solution of 75 mg (0.2 mmol) of [3-(3-fluoro-4-piperazin-1-ylphenyl)isoxazol-5-ylmethyl]isoxazol-3-ylamine (Intermediate 10) and 45 µL (0.24 mmol) of diisopropylamine in 2.5 mL of acetone, 36 µL (45 mg, 0.24 mmol) of phenoxyacetyl chloride were added. The mixture was stirred for 3 h at room temperature, and then treated with a 5% sodium bicarbonate solution. The organic layer was dried over anhydrous sodium sulfate, and filtered, and the solvent was distilled off under reduced pressure. The resulting crude product was chromatographed with dichloromethane/methanol gradient (100:0 to 50:1) as eluant to give 56 mg (yield = 53%) of a white solid corresponding to the title compound. IR (KBr): 3310, 1659 cm⁻¹. ¹H-NMR (300 MHz, d₆-DMSO, δ(ppm)): 8.41 (d, 1H), 7.66-7.60 (m, 2H), 7.28 (t, 2H), 7.12 (t, 1H), 6.95-6.91 (m, 3H), 6.87 (s, 1H), 6.04 (d, 1H), 4.85 (s, 2H), 4.44 (s, 2H), 3.64 (br, 4H), 3.12 (br, 2H), 3.06 (br, 2H).

### Example 8. Preparation of 3-[3-fluoro-4-(4-(1,2,4-triazol-1-yl)acetyl)piperazin-1-ylphenyl]-5-(isoxazol-3-ylaminomethyl)isoxazole

To a mixture of 200 mg (2.94 mmol) of 1,2,4-triazole, 811 mg (5.87 mmol) of anhydrous potassium carbonate, and 40 mL of dimethylsulfoxide, 600 mg (1.42 mmol) of 2-chloro-1-(4-{2-fluoro-4-[5-(isoxazol-3-ylaminomethyl)isoxazol-3-yl]phenyl}piperazin-1-yl)-ethanone (Intermediate 11) were added. The new mixture was stirred at room temperature for 48 h under inert atmosphere. The reaction mixture was diluted with 500 mL of ethyl acetate, washed four times with 200 mL of saturated sodium chloride solution, dried over anhydrous sodium sulfate and filtered. The solvent was distilled off under reduced pressure, and the obtained residue was chromatographed dn a silica gel column with dichloromethane/methanol (20:1) as eluant. Relevant fractions were combined and the solvent was evaporated. The obtained residue was broken up with diethyl ether, and filtered to give 10 mg (yield = 32%) of a yellow solid. IR(KBr): 3190 cm⁻¹, 1640 cm⁻¹. Mass spectrum (m/e): 452 (M⁺). ¹H-NMR (200 MHz, d₆-DMSO, δ(ppm)): 8.46 (s, 1H), 8.43 (d, 1H), 7.97 (s, 1H), 7.70-7.63 (m, 2H), 7.15 (t, 1H), 6.98-6.89 (m, 2H), 6.06 (d, 1H), 5.34 (s, 2H), 4.46 (d, 2H), 3.67 (m, 4H), 3.17 (m, 4H).

### Example 10. Preparation of 3-[3-fluoro-4-(1-pyrrolyl)acetyl)piperazin-1-ylphenyl]-5-(isoxazol-3-ylaminomethyl)isoxazole

To a mixture of 200 mg (2.94 mmol) of pyrrole, 811 mg (5.87 mmol) of anhydrous potassium carbonate, and 40 mL of dimethylsulfoxide, 600 mg (1.42 mmol) of 2-chloro-1-(4-{2-fluoro-4-[5-(isoxazol-3-ylaminomethyl)isoxazol-3-yl]phenyl}piperazin-1-yl)ethanone (Intermediate 11) were added. The new mixture was stirred for 36 h at room temperature under inert atmosphere. The reaction mixture was diluted with 500 mL of ethyl acetate, washed four times with 200 mL of saturated sodium chloride solution, dried over anhydrous sulfate, and filtered. The solvent was distilled off under reduced pressure. The obtained residue was purified by silica gel chromatography with dichloromethane/methanol (20:1) as eluant. Relevant fractions were combined and the solvent evaporated. The obtained residue was broken up with diethyl ether, and filtered to give 409 mg (yield = 63%) of a slightly yellow solid. IR (KBr): 3200, 1650 cm⁻¹. Mass spectrum (m/e): 450 (M⁺).

### Example 11. Preparation of 3-[3-fluoro-4-(3-pyridyloxyacetyl)piperazin-1-ylphenyl]-5-(isoxazol-3-ylaminomethyl)isoxazole

A solution of 20 mg (0.2 mmol) of 3-hydroxypiridine in 2 mL of tetrahydrofuran was treated with 10 mg sodium hydride (0.25 mmol, 60% oil dispersion) for 30 minutes at room temperature. Then, a solution of 75 mg (0.18 mmol) of 2-chloro-1-(4-{2-fluoro-4-[5-(isoxazol-3-ylaminomethyl)-isoxazol-3-yl]phenyl}piperazin-1-yl)ethanone (Intermediate 11) in 4 mL of tetrahydrofuran was added. The reaction mixture was stirred at 60°C for 24 h, and after cooling down, the precipitated solid was filtered off, and the solvent was distilled off at reduced pressure. The crude product was purified by column chromatography with dichloromethane/methanol gradient (100:0 to 50:1) to give 23 mg (yield = 26%) of an oily solid corresponding to the title compound. IR (KBr): 3322, 1657 cm⁻¹. ¹H-NMR (200 MHz, d₆-DMSO, δ(ppm)): 8.43 (d, 1H), 8.30 (m, 1H), 8.16 (m, 1H), 7.69-7.62 (m, 2H), 7.35 (m, 2H), 7.15 (t, 1H), 6.89 (s, 1H), 6.05 (d, 1H), 5.01 (s, 2H), 4.45 (s, 2H), 3.62 (m, 4H), 3.20 (m, 4H).

### Example 12. Preparation of 3-[13-fluoro-4-(2-pyridyloxyacetyl)piperazin-1-ylphenyl]-5-(isoxazol-3-ylaminomethyl)isoxazole

By an analogous process to that described in Example 11, 35 mg (yield = 41 %) of a whitish solid corresponding to the title compound were obtained. IR (KBr): 3322,1657 cm⁻¹.¹H-NMR (200 MHz, d₆-DMSO, δ(ppm)): 8.43 (d, 1H), 7.69-7.62 (m, 2H), 7.56 (dd, 1H), 7.44 (m, 2H), 7.16 (t, 1H), 6.89 (s, 1H), 6.38 (d, 1H), 6.22 (dt, 1H), 6.06 (d, 1H), 4.86 (s, 2H), 4.55 (s, 2H), 3.72 (m, 2H), 3.62 (m, 2H), 3.19 (m, 2H), 3.11 (m, 2H).

### Example 13. Preparation of 3-[3-fluoro-4-(3-nitrophenyloxyacetyl)piperazin-1-ylphenyl]-5-(isoxazol-3-ylaminomethyl)isoxazole

By an analogous process to that described in Example 11, 83 mg (yield = 89%) of a white solid corresponding to the title compound were obtained. IR (KBr): 3300,1657, 1527, 1350, 1233 cm⁻¹. ¹H-NMR (200 MHz, d₆-DMSO, δ(ppm)): 8.43 (d, 1H), 7.81 (dd, 1H), 7.76 (t, 1H), 7.69-7.62 (m, 2H), 7.57 (d, 1H), 7.42 (dd, 1H), 7.15 (t, 1H), 6.89 (s, 1H), 6.06 (d, 1H), 5.10 (s, 2H), 4.46 (s, 2H), 3.65 (br, 4H), 3.16 (br, 2H), 3.06 (br, 2H).

### Example 14. Preparation of 3-[3-fluoro-4-(4-nitrophenyloxyacetyl)piperazin-1-ylphenyl]-5-(isoxazol-3-ylaminomethyl)isoxazole

By an analogous process to that described in Example 11, 55 mg (yield = 55%) of a white solid corresponding to the title compound were obtained. IR (KBr): 3304, 1671, 1589, 1344, 1262 cm⁻¹. ¹H-NMR (200 MHz, d₆-DMSO, δ(ppm)): 8.43 (d, 1H), 8.21 (d, 1H), 7.69-7.62 (m, 2H), 7.17-7.13 (m, 3H), 6.89 (s, 1H), 6.06 (d, 1H), 5.14 (s, 2H), 4.46 (s, 2H), 3.63 (br, 4H), 3.16 (br, 2H), 3.06 (br, 2H).

### Example 15. Preparation of 3-[3-fluoro-4-(2-furylmethoxyacetyl)piperazin-1-ylphenyl]-5-(isoxazol-3-ylaminomethyl)isoxazole

By an analogous process to that described in Example 11, 26 mg (yield = 31 %) of a yellow solid corresponding to the title compound were obtained. IR (KBr): 3273, 1647 cm⁻¹. ¹H-NMR (200 MHz, d₆-DMSO, δ(ppm)): 8.43 (d, 1H), 7.67-7.61 (m, 3H), 7.12 (t, 1H), 6.88 (s, 1H), 6.45 (m, 1H), 6.05 (d, 1H), 4.49 (s, 2H), 4.45 (s, 2H), 4.20 (s, 2H), 3.57 (br, 4H), 3.06 (br, 4H).

### Example 16. Preparation of 3-[3-fluoro-4-(2-pyridylmethoxyacetyl)piperazin-1-ylphenyl]-5-(isoxazol-3-ylaminomethyl)isoxazole

By an analogous process to that described in Example 11, 63 mg (yield = 72%) of a white solid corresponding to the title compound were obtained. IR (KBr): 3256, 1639 cm⁻¹. ¹H-NMR (200 MHz, d₆-DMSO, δ(ppm)): 8.52 (dt, 1H), 8.43 (d, 1H), 7.83 (td, 1H), 7.69-7.61 (m, 2H), 7.48 (d, 1H), 7.28-7.34 (m, 1H), 7.12 (t, 1H), 6.89 (s, 1H), 6.06 (d, 1H), 4.63 (s, 2H), 4.45 (s, 2H), 4.36 (s, 2H), 3.61 (br, 4H), 3.10 (br, 4H).

### Example 17. Preparation of 3-[3-fluoro-4-(4-cyanophenoxyacetyl)piperazin-1-ylphenyl]-5-(isoxazol-3-ylaminomethyl)isoxazole

By an analogous process to that described in Example 11, 57 mg (yield = 61%) of a white solid corresponding to the title compound were obtained. IR (KBr): 3307, 2222, 1672 cm⁻¹. ¹H-NMR (200 MHz, d₆-DMSO, δ(ppm)): 8.43 (d, 1H), 7.84 (d, 2H), 7.69-7.61 (m, 2H), 7.18-7.09 (m, 3H), 6.89 (s, 1H), 6.05 (d, 1H), 5.06 (s, 2H), 4.45 (d, 2H), 3.63 (br, 4H), 3.11 (br, 4H).

### Example 18. Preparation of 3-[3-fluoro-4-(3-propyn-1-yloxyacetyl)piperazin-1-ylphenyl]-5-(isoxazol-3-ylaminomethyl)isoxazole

By an analogous process to that described in Example 11, 33 mg (yield = 43%) of a white solid corresponding to the title compound were obtained. IR (KBr): 3374, 3222, 2107, 1654 cm⁻¹. ¹H-NMR (200 MHz, d₆-DMSO, δ(ppm)): 8.43 (d, 1H), 7.69-7.61 (m, 2H), 7.13 (t, 1H), 6.95-6.89 (m, 2H), 6.06 (d, 1H), 4.46 (d, 2H), 4.26 (s, 2H), 4.24 (d, 2H), 3.59 (m, 4H), 3.51 (t, 1 H), 3.12 (br, 4H).

### Example 19. Preparation of 3-[3-fluoro-4-(4-fomylphenyloxyacetyl)piperazin-1-ylphenyl]-5-(isoxazol-3-ylaminomethyl)isoxazole

By an analogous process to that described in Example 11, 4 mg (yield = 4%) of a white solid corresponding to the title compound were obtained.

### Example 20: Preparation of 3-(3-fluoro-4-imidazol-1-ylphenyl)-5-(N-acetylaminomethyl)isoxazole

A mixture of 300 mg of imidazole (4.36 mmol), 40 mL of dimethylsulfoxide, 1.2 g (8.72 mmol) of anhydrous potassium carbonate, and 1 g (3.97 mmol) of *N*-[3-(3,4-difluorophenyl)isoxazol-5-ylmethyl]acetamide (Intermediate 9) was stirred at 90 °C for 24 h under inert atmosphere. After allowing to cool down, the reaction mixture was treated with 400 mL of saturated sodium chloride solution and extracted three times with 200 mL of ethyl acetate. The organic extract was dried over anhydrous sodium sulfate, and filtered, and the solvent was distilled off at reduced pressure. The obtained residue was purified by silica gel chromatography with dichloromethane/methanol as eluant. Relevant fractions were combined to give 220 mg (yield = 19%) of a white solid. Mass spectrum (m/e): 299 (M⁺). ¹H-NMR (200 MHz, CDCl₃, δ ppm): 8.00-6.90 (m, 5H), 6.0 (s, 1H), 4.40 (d, 2H), 2.1 (s, 3H).

### Example 21. Preparation of 3-(3-fluoro-4-imidazol-1-ylphenyl)-5-(N-thioacetylaminomethyl)isoxazole

A solution of 220 mg (0.733 mmol) of 3-(3-fluoro-4-imidazol-1-ylphenyl)-5-(*N-*acetylaminomethyl)isoxazole (Example 20) was suspended in 20 mL of dioxane. Then, 300 mg (0.733 mmol) of Lawesson reagent were added, and the mixture was refluxed under inert atmosphere with stirring for 1.5 h. After leaving the mixture 18 h at room temperature, the solvent was distilled off at reduced pressure. To the residue 300 mL of ethyl acetate were added, and the solution was washed twice with 200 mL of a saturated sodium chloride solution, dried over anhydrous sodium sulfate, and filtered. The solvent was evaporated under reduced pressure, and the obtained residue was purified by silica gel chromatography with dichloromethane/methanol (10:1) as eluant. The final product was broken up with diethyl ether to give 97 mg (yield = 42%) of a slightly yellow solid. Mass spectrum (m/e): 316 (M⁺). ¹H-NMR (200 MHz, CDCl₃, δ ppm): 8.05-6.85 (m, 5H), 5.90 (s, 1H), 4.30 (d, 2H), 2.2 (s, 3H).

### Example 22. Preparation of 1-(4-{2-fluoro-4-[5-(isoxazol-3-ylaminomethyl)-isoxazol-3-yl]-phenyl}piperazin-1-yl)-2-(quinolin-6-yloxy)ethanone

To a solution of 31 mg (0.213 mmol) of 6-hydroxyquinoline in 2 mL of *N,N*-dimethylformamide, 10 mg (0.250 mmol) of 60% sodium hydride in paraffin were added, and the suspension was stirred at room temperature for 2 h under inert atmosphere. Then, a solution of 75 mg (0.179 mmol) of 2-chloro-1-(4-{2-fluoro-4-[5-(isoxazol-3-ylaminomethyl)isoxazol-3-yl]-phenyl}-piperazin-1-yl)-ethanone (Intermediate 11) in 2 mL of *N,N*-dimethylformamide was added. The mixture was stirred at 50 °C for 10 h under inert atmosphere. After distilling off the solvent at reduced pressure, 6 mL of a mixture of dichloromethane/methanol (3:1) were added. The solvent was distilled off under reduced pressure, the residue was extracted three times with 2 mL of dichloromethane (3x2 mL), and the solvent was distilled off under reduced pressure. The residue was purified by silica gel chromatography with a dichloromethane/methanol gradient as eluant. Relevant fractions were combined to give 90 mg (yield = 95%) of a white solid. ¹H-NMR (200 MHz, d₆-DMSO. δ(ppm)): 8.81 (dd,1H), 8.10-8.02 (m, 3H), 7.53-7.34 (m, 5H), 7.20 (d, 1H), 6.50 (s, 1H), 5.95 (d1H), 4.88 (s, 2H), 4.62-4.55 (m, 2H), 3.83 (m, 4H), 3.12 (m, 4H).

### Example 23. Preparation of (1-{2-fluoro-4-[5-(isoxazol-3-ylaminomethyl)-isoxazol-3-yl]phenyl}-1H-pyrrol-3-yl)methanol

A solution of 1.0 g (2.82 mmol) of (1-{2-fluoro-4-[5-(isoxazol-3-ylaminomethyl)isoxazol-3-yl]-phenyl}-1*H*-pyrrol-3-carboxaldehyde (Example 5) in 48 mL of a mixture of methanol/dichloromethane (2:1) was cooled to 0°C, and 53.4 mg (1.41 mmol) of sodium borohydride were added. After stirring for 4 h at room temperature the mixture was diluted with 250 mL of dichloromethane, and washed with water. The organic layer was dried over anhydrous sodium sulfate, and the solvent was distilled off under reduced pressure to give 735 mg (yield = 73%) of a yellow solid. ¹H-NMR (200 MHz, CDCl₃, δ(ppm)): 8.44 (d, 1H), 7.96-7.63 (m, 3H), 7.19-7.14 (m, 2H), 7.01 (s, 1H), 6.95 (t, 1H), 6.29 (dd, 1H), 6.06 (d, 1H), 4.83 (t1 H), 4.50 (d, 2H), 4.39 (d, 2H).

### Example 24. Preparation of (1-{2-fluoro-4-[5-(isoxazol-3-ylaminomethyl)-isoxazol-3-yl]phenyl}-1H-pyrrol-3-carboxaldehyde oxime

To a mixture of 1.0 g (2.82 mmol) of (1-{2-fluoro-4-[5-(isoxazol-3-ylaminomethyl)isoxazol-3-yl]phenyl}-1*H*-pyrrol-3-carboxaldehyde (Example 5), and 0.25 g of potassium carbonate in 70 mL of methanol/dichloromethane (1:1), 0.25 g (3.57 mmol) of hydroxylamine hydrochloride were added. The mixture was stirred for 48 h at room temperature, diluted with ethyl acetate, washed with water, dried over anhydrous sodium sulfate, and filtered. The solvent was distilled off under reduced pressure. The residue was purified by silica gel chromatography with a dichloromethane/methanol gradient as eluant. Relevant fractions were combined to give 100 mg (yield = 10%) of a white solid. ¹H-NMR (200 MHz, d₆-DMSO, δ(ppm)): 10.65 (s, 1H), 8.44 (d, 1H), 8.04-7.70 (m, 4H), 7.51 (d, 1 H), 7.30 (d, 1H), 7.03 (s, 1H), 6.95 (t, 1H), 6.54 (m, 1 H), 6.06 (d, 1 H), 4.50 (d, 1 H).

### Example 25. Preparation of (1-{2-fluoro-4-[5-(isoxazol-3-ylaminomethyl)isoxazol-3-yl]phenyl}1H-pyrrol-3-nitrile

To a solution of 200 mg (0.55 mmol) of (1-{2-fluoro-4-[5-(isoxazol-3-ylaminomethyl)isoxazol-3-yl]phenyl}1*H*-pyrrol-3-carboxaldehyde oxime (Example 24) in 15 mL acetonitrile/carbon tetrachloride (1:1), 600 mg (2.29 mmol) of triphenylphosphine were added, and the mixture was refluxed for 8 h with stirring. The mixture was diluted with 100 mL of ethyl acetate, and filtered. The solvent was distilled off under reduced pressure. The obtained residue was broken up with diethyl ether to give 57 mg (yield = 30%) of a white solid. ¹H-NMR (200 MHz, CDCl₃, δ(ppm)): 8.11 (d, 1H), 7.75-7.65 (m, 2H), 7.54-7.43 (m, 2H), 7.04 (m, 1H), 6.64-6.61 (m, 2H), 5.91 (d, 1H), 4.65 (d, 2H), 4.47(t, 1H).

### Example 26. Preparation of 4-[2-(4-{2,6-difluoro-4-[5-(isoxazol-3-ylaminomethyl)isoxazol-3-yl]phenyl}piperazin-1-yl)-2-oxoethoxy]benzaldehyde

To a solution of 45 mg (0.250 mmol) of 4-formylphenoxyacetic acid in 2 mL of dichloromethane, 49 mg (0.412 mmol) of thionyl chloride were added, and the mixture was stirred for 2 h at room temperature. The solvent was distilled off under reduced pressure, and the residue was dissolved again with 2 mL of dichloromethane, and then evaporated. 75 mg (0.207 mmol) of [3-(3,5-difluoro-4-piperazire-1-ylphenyl)isoxazol-5-ylmethyl]isoxazol-3-ylamine (intermediate 18), and 43 µL (0.333 mmol) of *N*,*N*-diisopropylethylamine were added. The mixture was stirred for 20 h at room temperature, and filtered. The solvent was distilled off under reduced pressure. The crude product was purified by silica gel chromatography with a dichloromethane/methanol gradient (100:0 to 98:2) as eluant to give 25 mg (yield = 23%) of a white solid corresponding to the title compound. ¹H-NMR (200 MHz, d₆-DMSO, δ(ppm)): 9.88 (s, 1H), 8.40 (d, 1H), 7.86 (d, 2H), 7.57 (s, 1H), 7.54 (s, 1H), 7.13 (d, 2H), 6.93 (s, 1H), 6.82 (t, 1H), 6.04 (d, 1H), 5.04 (s, 2H), 4.47 (d, 2H), 3.60 (m, 4H), 3.24 (m, 4H).

### Example 27. Preparation of 1-{2-fluoro-4-[5-(isoxazol-3-ylaminomethyl)isoxazol-3-yl]phenyl}-1H-imidazol-4-carboxaldehyde

By an analogous process to that described in Example 2, 450 mg (yield = 18%) of a yellow solid corresponding to the title compound were obtained. ¹H-NMR (200 MHz, d₆-DMSO, δ(ppm)): 9.85 (s, 1H), 8.49 (s, 1H), 8.41 (d, 1H), 8.31 (s, 1H), 8.03 (d, 1H), 7.90-7.88 (m, 2H), 7.04 (s, 1H), 6.86 (s, 1 H), 6.06 (d, 1 H), 4.51 (d, 2H).

### Example 28. Preparation of 3-(1-{2-fluoro-4-[5-(isoxazol-3-ylaminomethyl)isoxazol-5-yl]phenyl}-1H-imidazol-4-yl)acrylonitrile

To a solution of 354 mg (2.00 mmol) of diethylcyanomethyl phosphonate in 10 mL of tetrahydrofuran, 156 mg (1.39 mmol) of potassium *tert*-butoxide were added. The mixture was stirred for five minutes at room temperature, and then added over a suspension of 353 mg (1.00 mmol) of 1-{2-fluoro-4-[5-{isoxazol-3-ylaminomethyl)isoxazol-3-yl]phenyl}-1*H*-imidazol-4-carboxaldehyde (Example 27) in 10 mL of tetrahydrofuran. The new mixture was stirred for 4 h at room temperature, diluted with 50 mL of a 5% sodium bicarbonate solution, and extracted with dichloromethane. The organic layer was dried over anhydrous sodium sulfate, and filtered, and the solvent was distilled off under reduced pressure. The resulting residue was broken up with diethyl ether to give 232 mg (yield = 62%) of a yellow solid. ¹H-NMR (200 MHz, CDCl₃/d₆-DMSO, δ(ppm)): 8.43 (s, 1H), 8.27-7.84 (m, 5H), 7.57 (d, 1H), 7.07 (s, 1H), 6.97 (t1 H), 6.19 (d,1 H), 6.07 (d, 1 H), 4.51 (d, 2H).

### Example 29. Preparation of [3-(3-fluoro-4-[(2-methoxiphenylamino)-methyl]imidazol-1-yl}phenyl)isoxazol-5-ylmethyl]isoxazol-3-ylamine

A solution of 75 mg (0.212 mmol) of 1-{2-fluoro-4-[5-(isoxazol-3-ylaminomethyl)isoxazol-3-yl]phenyl}-1*H*-imidazol-4-carboxaldehyde (Example 27), and 26.1 mg (0.212 mmol) of 2-methoxyphenylamine in 2 mL of dichloromethane/trimethyl ortoformiate (1:1) was stirred at 25°C for 20 h. The solvent was distilled off under reduce pressure. The [3-(3-fluor-4-{4-[(2-mothoxi-phenyliminio)-methyl]imidazol-yl)phenyl)isoxazol-5-ylmethyl]isoxazol-3-ylamine obtained was dissolved in 4 mL of dichloromethane. To the solution, 100 mg (0.472 mmol) of sodium triacetoxyborohydride were added. The mixture was stirred at room temperature for 20 h, and then 1 mL of deionized water was added. After distilling off the solvent, 2 mL of methanol were added and evaporated under reduced pressure. After repeating the last step three times, the product was dissolved in dichloromethane, and was dried passing the solution through anhydrous sodium sulfate. The solvent was evaporated, and the obtained residue was purified by silica gel chromatography with a dichloromethane/methanol gradient as eluant. Relevant fractions were combined to give 90 mg (yield = 51 %) of a brown pasty solid. ¹H-NMR (200 MHz, d₆-DMSO, δ(ppm)): 8.44 (d, 1H), 8.10-7.78 (m. 5H), 7.51 (s, 1H), 7.08-6.53 (m, 6H), 6.06 (d, 1H), 5.21 (t. 1H), 4.50 (d. 2H), 4.22 (d, 2H), 3.79 (s, 3H).

### Example 30. Preparation of (3-{3-fluoro-4-[3-(o-tolylaminomethyl)pyrrol-1-yl]-phenyl]isoxazol-5-ylmethyl)isoxazol-3-ylamine

By an analogous process to that described in Example 29, 41.4 mg (yield = 33%) of a yellow solid corresponding to the title compound were obtained. ¹H-NMR (200 MHz, d₆-DMSO, δ(ppm)): 8.44 (d, 1 H), 7.92 (dd, 1H) 7.82 (dd, 1H), 7.67 (t, 1H), 7.21 (m, 2H), 7.01-6.93 (m, 3H), 6.62 (d, 1H), 6.49 (t, 1H), 6.35 (dd, 1H), 6.06 (d, 1 H), 5.09 (t, 1H), 4.49 (d, 2H), 4.20 (d, 2H), 2.06 (s, 3H).

### Example 32. Preparation of [3-(3-fluoro-4-imidazol-1-ylphenyl)isoxazol-5-ylmethyl]-(3-methylisotiazol-5-yl)amine

By an analogous process to that described in Example 2, 10 mg (yield = 1%) of a yellow solid corresponding to the title compound were obtained.

### Example 33. Preparation de 1-(2-fluoro-4-{5-[(3-methylisotiazol-5-ylamino)-methyl]isoxazol-3-yl]}phenyl)-1H-imidazol-4-carboxaldehyde

By an analogous process to that described in Example 2, 11 mg (yield = 1%) of a yellow solid corresponding to the title compound were obtained. ¹H-NMR (200 MHz, CDCl₃, δ(ppm)): 9.99 (s, 1H), 7.98 (t, 1H), 7.94 (t, 1H), 7.80 (dd, 1H), 7.76-7.73 (m, 1H), 7.54 (t, 1H), 6.59 (s, 1H), 6.12 (s, 1H), 5.02 (t, 1H), 4.56 (t, 2H), 2.33 (s, 3H).

### Example 34, Preparation de {3-[3-fluoro-4-(4-methylimidazol-yl)phenyl]isoxazol-5-ylmethyl}-(3-methyl-isothiazol-5-yl)amine

By an analogous process to that described in Example 2, 41 mg (yield = 3%) of a yellow solid corresponding to the title compound were obtained. ¹H-NMR (200 MHz, CDCl₃, δ(ppm)): 7.80 (s, 1H), 7.74-7.64 (m, 2H), 7.46 (t, 1H), 7.03 (s, 1H), 6.56 (s, 1H), 6.12 (s, 1H), 5.15 (t, 1H), 4.55 (d, 2H), 2.33 (s, 3H), 2.32 (s, 3H).

### Example 35. Preparation of 1-{2-fluoro-4-[5-(isoxazol-3-yloxymethyl)isoxazol-3-yl]phenyl}-1-H-imidazol-4-carboxaldehyde

By an analogous process to that described in Example 2, 41 mg (yield = 3%) of a yellow solid corresponding to the title compound were obtained. ¹H-NMR (200 MHz, d₆-DMSO, δ(ppm)): 9.86 (s, 1H), 8.75 (s, 1H), 8.55 (s, 1H), 8.36 (s, 1H), 8.13-7.95 (m, 3H), 7.40 (s, 1H), 6.46 (s, 1H), 5.53 (s, 2H).

## Claims

1. A (3,5)-disubstituted isoxazolinic type compound of formula (I), 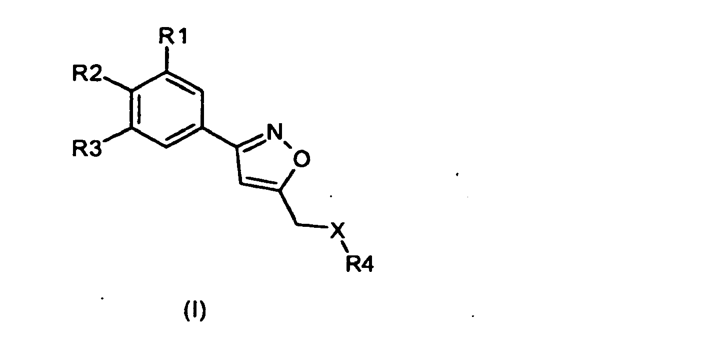 stereoisomers, mixtures of stereoisomers, polymorphic forms, mixtures of polymorphic forms, *N*-oxides, solvates and pharmaceutically acceptable salts thereof, wherein
X is a biradical selected from the group consisting of O, S, NH, OCO, NH-CO, NH-COO, NH-CS, NH-CO-NH and NH-CS-NH;
R4 is a radical selected from the group consisting of:
- hydrogen,
- (C₁-C₃)-alkyl, optionally substituted by 1, 2 or 3 halogen radicals selected from F, Cl or Br; and
- a *C*-linked heterocyclic radical HET1 that is:
either a *C*-linked radical of a 5-membered heterocycle of 1, 2, 3 or 4 heteroatoms selected from the group consisting of N, O and S, optionally substituted by a radical selected from the group consisting of (C₁-C₄)-alkyl, amino, (C₁-C₄)-alkylamino, (C₁-C₄)-alkoxyl, (C₁-C₄)-alkoxycarbonyl, (C₁-C₄)-alkylcarbonyl, (C₁-C₄)-amido, amido, CN, NO₂, F, CI, and Br;
or a *C*-linked radical of a 6-membered heterocycle with 1, 2 or 3 atoms of nitrogen, optionally substituted by 1, 2 or 3 substituents independently selected from the group consisting of (C₁-C₄)-alkyl, amino, (C₁-C₄)-alkylamino, (C₁-C₄)-alkoxyl, (C₁-C₄)-alkoxycarbonyl, (C₁-C₄)-alkylcarbonyl, (C₁-C₄)-amido, amido, CN, NO₂, F, CI and Br;
R1 and R3 each independently represent H or F;
R2 is an *N*-linked or *C*-linked radical selected from the group consisting of: 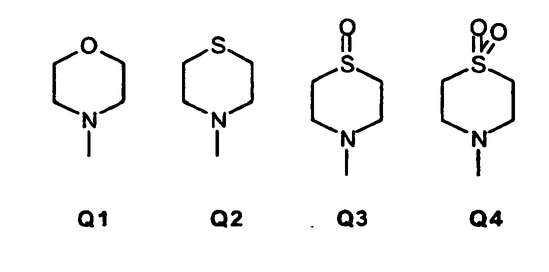 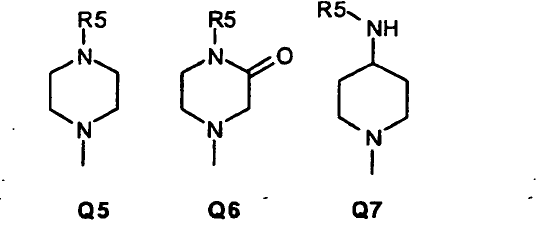 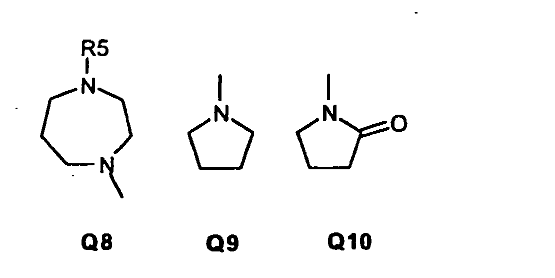 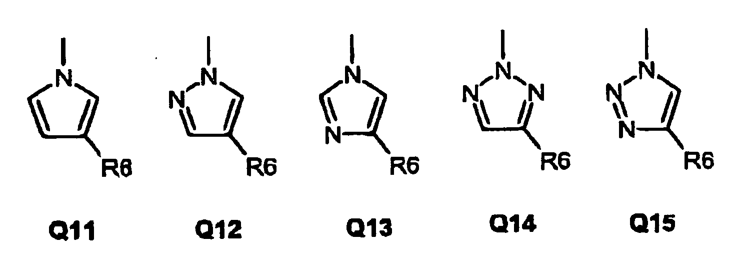 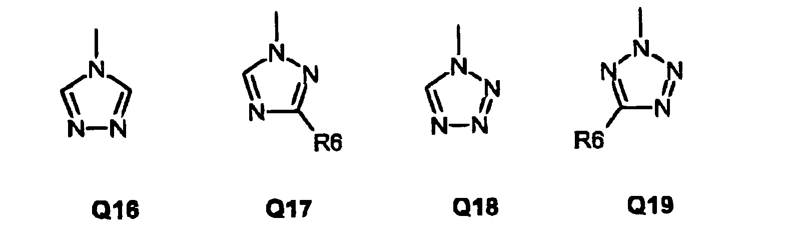 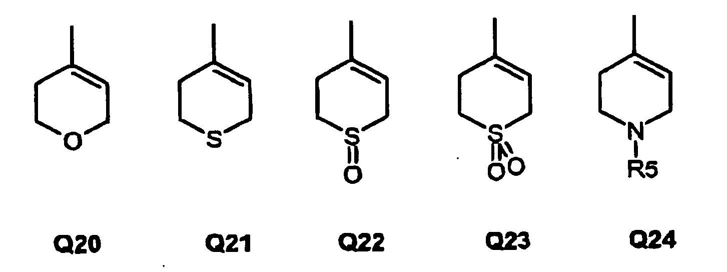 wherein:
R5 is a non cyclic radical selected from the group consisting of:
-(CH₂)ₙ-CO-R7,
and SO₂-R7
wherein:
R7 is (C₁-C₄)-alkyl, (C₁-C₃)-alkenyl (straight or branched), -(CH₂)ₚ-R2, -(CH₂)ₘ-Y-(CH₂)_{q} -R8 or HET2;
n, p, q and m are integers from 0 to 8;
Y is O, S or NH;
R2 is as defined above, excluding Q20, Q21, Q22, Q23 and Q24.
R8 is H or a C-linked radical selected from the group consisting of (C₁-C₃)-alkyl, vinyl, allyl, ethinyl, propargyl, phenyl, phenyl substituted by CHO, CN, NO₂, CH₃ or F and a C-linked radical of an aromatic system constituted by a 5- or 6-membered ring, or by two 5- or 6-membered fused rings; containing the aforementioned aromatic system from one to three heteroatoms independently selected from O, N and S; and being the aforementioned aromatic system optionally mono-, di- or trisubstituted by radicals independently selected from the group consisting of H, (C₁-C₄)-alkyl (straight or branched), (C₁-C₄)-alkoxyl, (C₁-C₄)-alkylsulfanyl, NHCO-R9, NHCOO-R9, CO-R9, COO-R9, CN, NO, NO₂, CH=N-R10, F, Cl and Br or said aromatic system being selected from the group consisting of 2-, 3- and 4-pyridyl all of which may optionally be substituted by CHO, CN, NO₂, CH₃ or F ;
R9 is H, (C₁-C₃)-alkyl or N(R11)(R12), wherein R11 and R12 are independently selected from the group consisting of H and (C₁-C₃)-alkyl;
R10 is H, (C₁-C₃)-alkyl, phenyl, benzyl, OH or (C₁-C₃)-alkyloxy;
HET2 is a C-linked heterocyclic radical selected from the group consisting of: 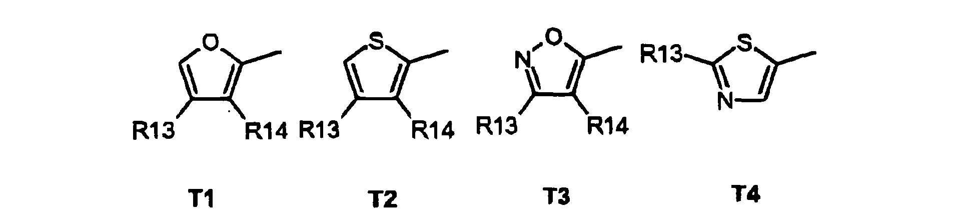 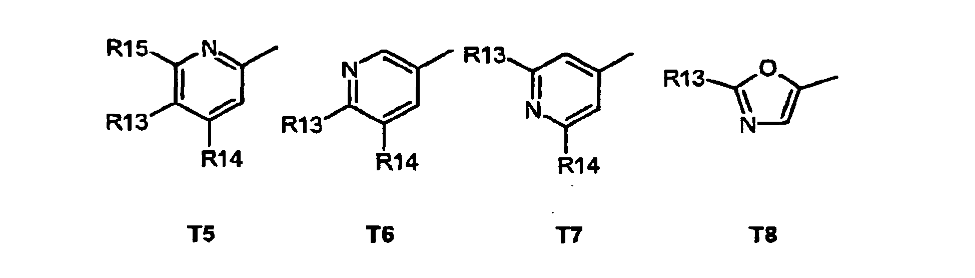 wherein R13, R14 and R15 are radicals independently selected from the group consisting of (C₁-C₄)-alkyl (straight or branched), (C₁-C₄)-alkoxyl, (C₁-C₄)-alkylsulfanyl, NHCO-R9, NHCOO-R9, CO-R9, COO-R9, CN, NO, NO₂, CH=N-R10, F, Cl and Br, wherein R9 and R10 are as defined above;
alternatively, R5 is C-linked heterocyclic radical selected from the group consisting of: 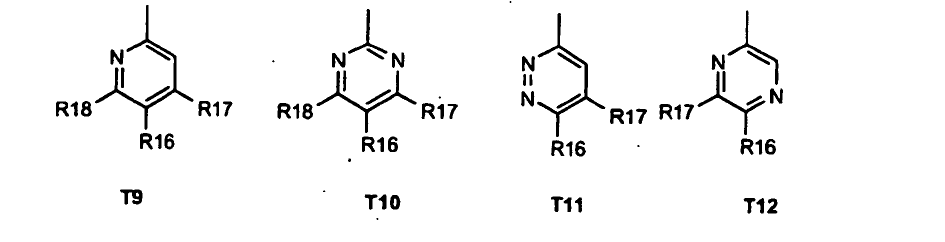 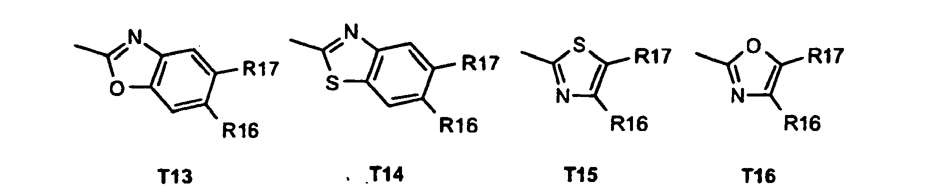 wherein R16, R17 and R18 are independently selected from the group consisting of CO-R9, COO-R9, CN, NO, NO₂, and CH=N-R10;
R6 is selected from the group consisting of H, F, Cl, Br, trifluoromethyl, CN, NO₂, CHO, CH₂OH, (C₁-C₃)-alkyl, (C₁-C₃)-alkoxyl, (C₁-C₃)-alkoxycarbonyl, (C₁-C₃)-alkoxy-(C₁-C₃)-alkyl, benzyloxy-(C₁-C₃)-alkyl, (C₁-C₃)-alkylcarbonyl, CO-NR19R20, NR19R20, (C₁-C₃)-alkylamino, (C₁-C₃)-alkyl-CH=N-O-R21 , CH=N-O-R21, CH=CR22R23, (CH₂)ₛNHR19, and CH=NR19; wherein R19 and R20, are independently selected from the group consisting of H, (C₁-C₃)-alkyl, CO-R24 and an aromatic system constituted by a 5- or 6-membered ring, or by two 5- or 6-membered fused rings; optional containing the aforementioned aromatic system from one to three heteroatoms independently selected from the group consisting of O, N and S; and being the aforementioned aromatic system optionally mono-, di- or trisubstituted by radicals independently selected from the group consisting of H, (C₁-C₄)-alkyl (straight or branched), (C₁-C₄)-alkoxyl, (C₁-C₄)-alkylsulfanyl, NHCO-R9, NHCOO-R9, CO-R9, COO-R9, CN, NO, NO₂, CH=N-R10, F, CI and Br; R21 is H or (C₁-C₃)-alkyl; R22 and R23, are independently selected from the group consisting of H, CN, NO₂, (C₁-C₃)-alkylcarbonyl, (C₁-C₃)-alkoxycarbonyl, CHO, CONR19R20 and CH₂OH; and R24 is H, (C₁-C₃)-alkyl, (C₁-C₃)-alkoxyl or HET2, wherein HET2 is as defined above; s is a integer comprised from 0 to 4.

2. The compound according to claim 1, wherein:
X is NH or NH-CS;
R4 is methyl or a C-linked isoxazole or isothiazole radical optionally substituted by a methyl moiety in any of their substitutable positions;
R1 is H and R3 is F;
R2 is a radical selected from the group consisting of: 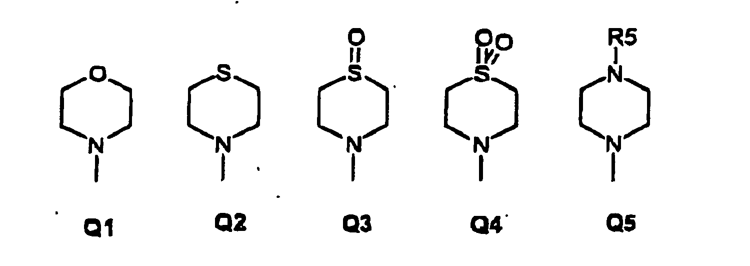 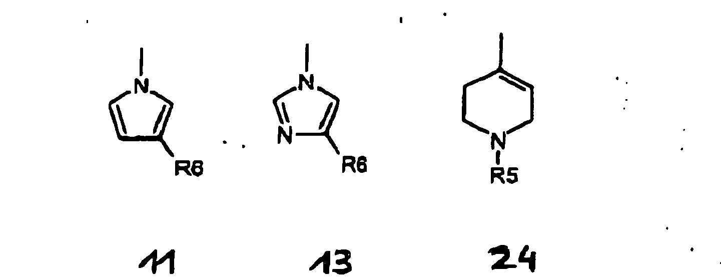
R5 is CO-R7;
R7 is selected from (CH₂)ₘ-Y-R8 and HET2, wherein m =1;
Y is O or NH;
R8 is selected from the group consisting of H, phenyl and 2-, 3-, 4-pyridyl, being the last four optionally substituted by CHO, CN, NO₂, CH₃ or F;
HET2 is selected from the group consisting of: 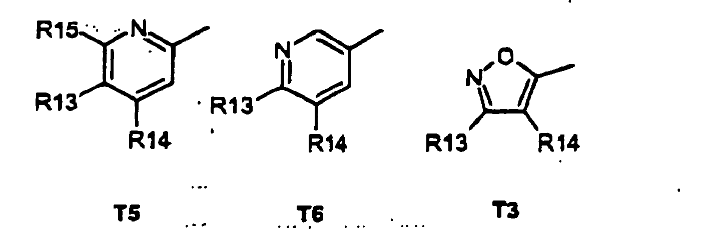 wherein R13, R14 and R15 are independently selected from the group consisting of CN, NO₂ and CHO;
and R6 is selected from the group consisting of H, CH₃, CN, CHO, CH₂OH, CH=N-OH, CH=CHCN, CO-CH₃ and CH₂NH-phenyl, said phenyl being substituted by a radical selected from the group consisting of F, CN, CHO and NO₂.

3. The compound according to any of the claims 1 or 2, for use in the therapeutic treatment of human or animal body.

4. The compound according to any of the claims 1 or 2, for use in the treatment of microbial infections.

5. The compound according to claim 4, wherein the treatment is carried out by oral, parenteral, or topical administration.

6. Use of the compound defined in any of the claims 1 or 2, for the manufacture of a medicament for the treatment of microbial infections.

7. Use according to claim 6, wherein the medicament can be administered by oral, parenteral or topical route.

8. The compound according to any of the claims 1 or 2, for use in the treatment of cancerous and precancerous pathologies.

9. The compound according to claim 8, wherein the treatment is carried out by oral, parenteral, or topical administration.

10. Use of the compound defined in any of the claims 1 or 2, for the manufacture of a medicament for the treatment of cancerous and precancerous pathologies.

11. Use according to claim 10, wherein the medicament is administered by oral, parenteral or topical route.

12. A pharmaceutical composition comprising a therapeutically effective amount of a compound as defined in any of the claims 1 or 2, and pharmaceutically acceptable excipients or solvents.

## Patentansprüche

1. (3,5)-Disubstituerte Verbindung vom Isoxazolin-Typ mit der Formel (I), 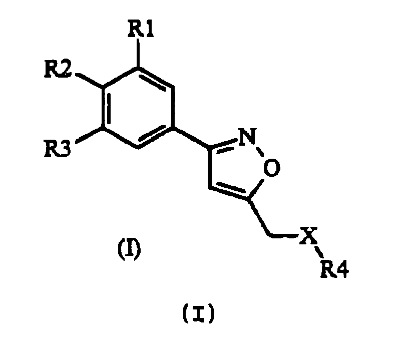 Stereoisomere, Mischungen von Stereoisomeren, polymorphe Formen, Mischungen von polymorphen Formen, N-Oxide, Solvate und pharmazeutisch annehmbare Salze davon, worin
X ein Biradikal ist, ausgewählt aus der Gruppe bestehend aus O, S, NH, OCO, NH-CO, NH-COO, NH-CS, NH-CO-NH und NH-CS-NH;
R4 ein Rest ist, ausgewählt aus der Gruppe bestehend aus:
- Wasserstoff,
- (C₁-C₃)-Alkyl, wahlweise substituiert mit 1, 2 oder 3 Halogenresten, ausgewählt unter F, Cl oder Br; und
- einem C-gebundenen heterocyclischen Rest HET1, welcher ist:
entweder ein C-gebundener Rest eines 5-gliedrigen Heterocyclus mit 1, 2, 3 oder 4 Heteroatomen, ausgewählt aus der Gruppe bestehend aus N, O und S, wahlweise substituiert mit einem Rest, ausgewählt aus der Gruppe bestehend aus (C₁-C₄)-Alkyl, Amino, (C₁-C₄) -Alkylamino, (C₁-C₄) -Alkoxyl, (C₁-C₄) - Alkoxycarbonyl, (C₁-C₄)-Alkylcarbonyl, (C₁-C₄)-Amido, Amido, CN, NO₂, F, Cl und Br;
oder ein C-gebundener Rest eines 6-gliedrigen Heterocyclus mit 1, 2 oder 3 stickstoffatomen, wahlweise substituiert mit 1, 2 oder 3 Substituenten, unabhängig ausgewählt aus der Gruppe bestehend aus (C₁-C₄)-Alkyl, Amino, (C₁-C₄)-Alkylamino, (C₁-C₄)-Alkoxyl, (C₁-C₄)-Alkoxycarbonyl, (C₁-C₄) -Alkylcarbonyl, (C₁-C₄)-Amido, Amido, CN, NO₂, F, Cl und Br;
R₁ und R₃ jeweils unabhängig H oder F repräsentieren;
R₂ ein N-gebundener oder C-gebundener Rest ist, ausgewählt aus der Gruppe bestehend aus: 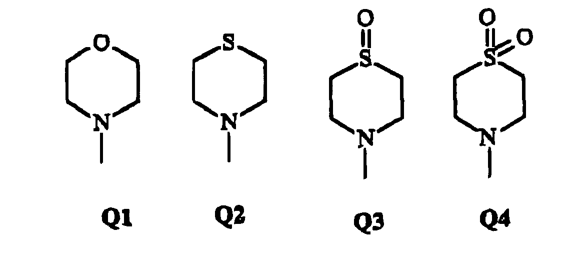 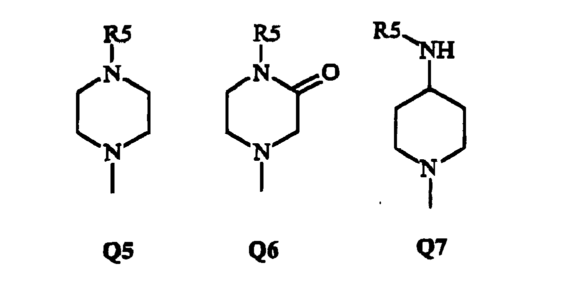 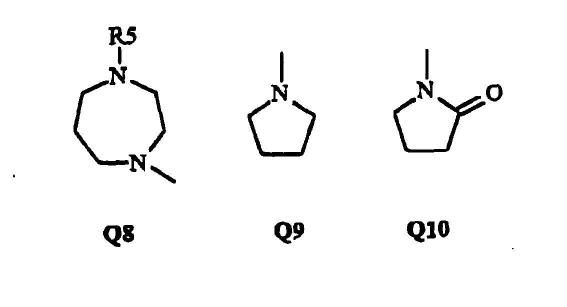 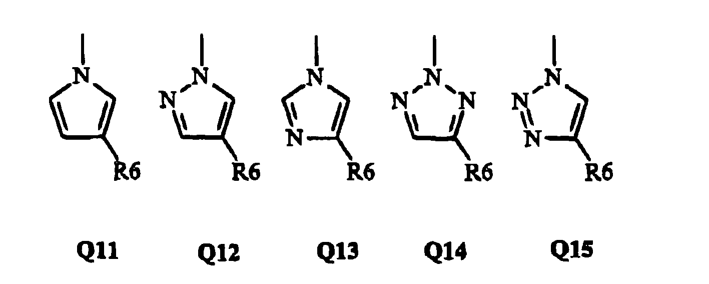 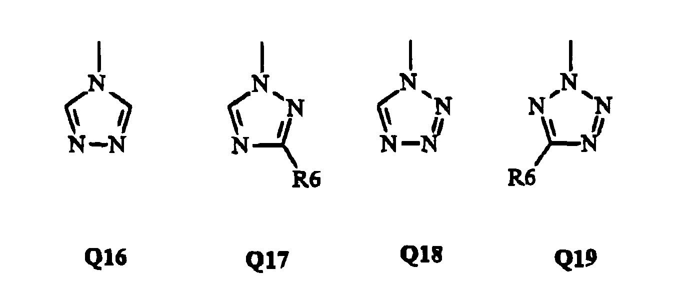 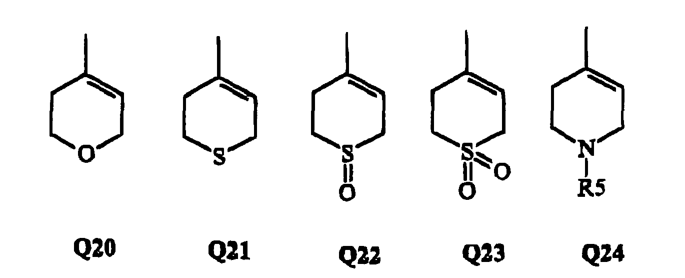 worin:
R5 ein nichtcyclischer Rest ist, ausgewählt aus der Gruppe bestehend aus:
**-** (CH₂)ₙ-CO-R7
und SO₂-R7
worin:
R7 (C₁-C₄) -Alkyl, (C₁-C₃) -Alkenyl (gerade oder verzweigt), - (CH₂)ₚ-R2, - -(CH₂)ₘ-Y-(CH₂)_{q}-R8 oder HET2 ist;
n, p, q und m ganze Zahlen zwischen 0 und 8 sind;
Y O S oder NH ist;
R2 wie oben definiert ist, ausgenommen Q20, Q21, Q22, Q23 und Q24;
R8 H oder ein C-gebundener Rest ist, ausgewählt aus der Gruppe bestehend aus (C₁-C₃)-Alkyl, vinyl, Allyl, Ethinyl, Propargyl, Phenyl, Phenyl substituiert mit CHO, CN, NO₂, CH₃ oder F und einem C-gebundenen Rest eines aromatischen Systems, das von einem 5- oder 6-gliedrigen Ring oder von zwei 5- oder 6- gliedrigen kondensierten Ringen gebildet wird; wobei das vorgenannte aromatische System ein bis drei Heteroatome enthält, die unabhängig unter O, N und S ausgewählt sind; und wobei das vorgenannte aromatische System wahlweise mit Resten mono-, di- oder trisubstituiert ist, die unabhängig aus der Gruppe bestehend aus H, (C₁-C₄)-Alkyl (gerade oder verzweigt), (C₁-C₄)-Alkoxyl, (C₁-C₄)-Alkyloulfanyl, NHCO-R9, NHCOO-R9, CO-R9, COO-R9, CN, NO, NO₂, CH=N-R10, F, Cl und Br oder das vorgenannte aromatische System aus der Gruppe bestehend aus 2-, 3- und 4-Pyridyl ausgewählt ist, wobei sie wahlweise mit CHO, CN, NO2, CH3 oder F substituiert sind;
R9 H, (C₁-C₃) -Alkyl oder N(R11) (R12) ist, worin R11 und R12 unabhängig aus der Gruppe bestehend aus H und (C₁-C₃)-Alkyl ausgewählt sind;
R10 H, (C₁-C₃)-Alkyl, Phenyl, Benzyl, OH oder (C₁-C₃)-Alkyloxy ist;
HET2 ein C-gebundener heterocyclischer Rest ist, ausgewählt aus der Gruppe bestehend aus: 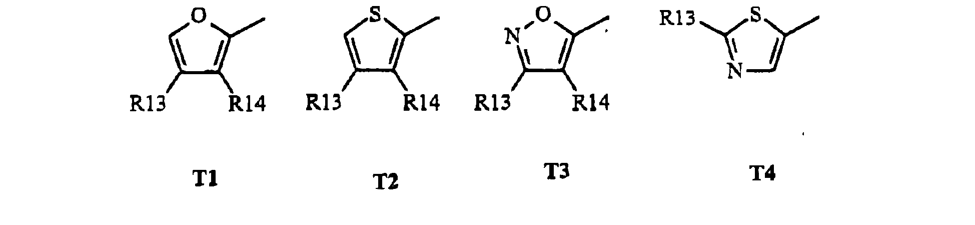 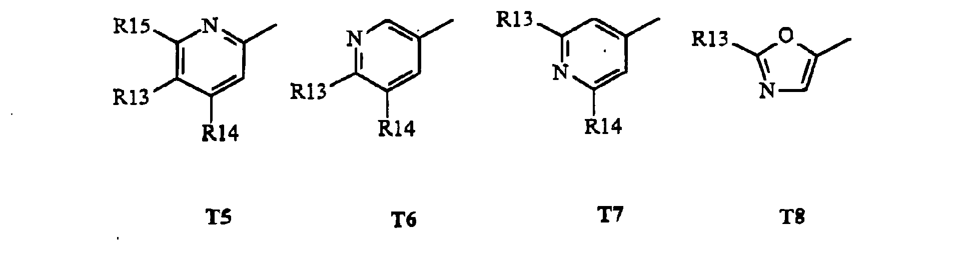 worin R13, R14 und R15 Reste sind, die unabhängig aus der Gruppe bestehend aus (C₁-C₄)-Alkyl (gerade oder verzweigt), (C₁-C₄)-Alkoxyl, (C₁-C₄)-Alkylsulfanyl, NHCO-R9, NHCOO-R9, CO-R9, COO-R9, CN, NO, NO₂, CH=N-R10, F, Cl und Br ausgewählt sind, worin R9 und R10 wie oben definiert sind;
R5 alternativ ein C-gebundener heterocyclischer Rest ist, ausgewählt aus der Gruppe bestehend aus: 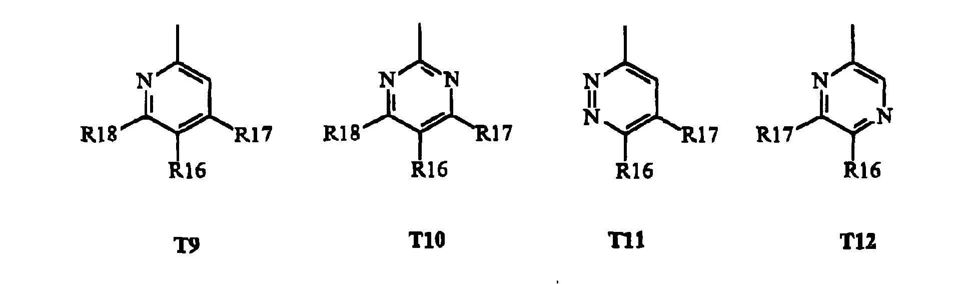 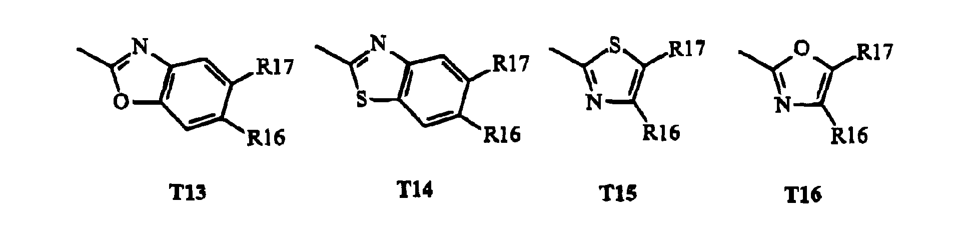 worin R16, R17 und R18 unabhängig aus der Gruppe bestehend aus CO-R9, COO-R9, CN, NO, NO₂ und CH=N-R10 ausgewählt sind;
R6 aus der Gruppe bestehend aus H, F, Cl, Br, Trifluormethyl, CN, NO₂, CHO, CH₂OH, (C₁-C₃)-Alkyl, (C₁-C₃)-Alkoxyl, (C₁-C₃)-Alkoxycarbonyl, (C₁-C₃)-Alkoxy-(C₁-C₃)-alkyl, Benzyloxy-(C₁-C₃)-alkyl, (C₁-C₃)-Alkylcarbonyl, CO-NR19R20, NR19R20, (C₁-C₃)-Alkylamino, (C₁-C₃)-Alkyl-CH=N-O-R21, CH=N-O-R21, CH=CR22R23, (CH₂)₈NHR19 und CH=NR19 ausgewählt ist; worin R19 und R20 unabhängig aus der Gruppe bestehend aus H, (C₁-C₃) -Alkyl, CO-R24 und einem aromatischen System, das von einem 5- oder 6- gliedrigen Ring oder von zwei 5- oder 6-gliedrigen kondensierten Ringen gebildet wird, ausgewählt sind; wobei das vorgenannte aromatische System ein bis drei Heteroatome enthält, die unabhängig aus der Gruppe bestehend aus O, N und S ausgewählt sind; und wobei das vorgenannte aromatische System wahlweise mit Resten mono-, di- oder trisubstituiert ist, die unabhängig aus der Gruppe bestehend aus H, (C₁-C₄) -Alkyl (gerade oder verzweigt), (C₁-C₄)-Alkoxyl, (C₁-C₄)-Alkylsulfanyl, NHCO-R9, NHCOO-R9, CO-R9, COO-R9, CN, NO, NO₂, CH=N-R10, F, Cl und Br ausgewählt sind; R21 H oder (C₁-C₃)-Alkyl ist; R22 und R23 unabhängig aus der Gruppe bestehend aus H, CN, NO₂, (C₁-C₃)-Alkyloarbonyl, (C₁-C₃) -Alkoxycarbonyl, CHO, CONR19R20 und CH₂OH ausgewählt sind; und R24 H, (C₁-C₃)-Alkyl, (C₁-C₃)-Alkoxyl oder HET2 ist, worin HUT2 wie oben definiert ist; s eine ganze Zahl zwischen 0 und 4 ist.

2. Verbindung nach Anspruch 1, worin:
X NH oder NH-CS ist;
R4 Methyl oder ein C-gebundener Isoxazol- oder Isothiazolrest ist, der wahlweise an irgendeiner seiner substituierbaren Positionen mit einem Methylanteil substituiert ist;
R1 H und R3 F ist;
R2 ein Rest ist, ausgewählt aus der Gruppe bestehend aus: 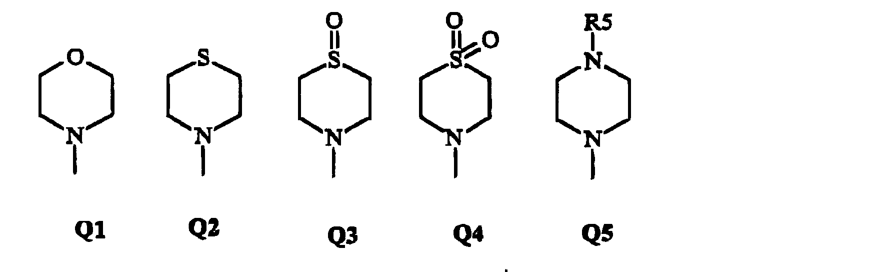 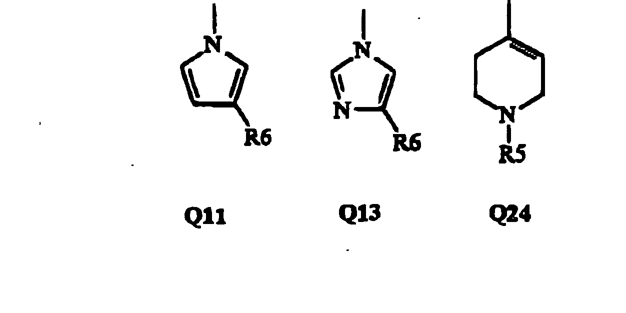
R5 CO-R7 ist;
R7 unter (CH₂)ₘ-Y-R8 und HET2 ausgewählt ist, worin m = 1;
Y O oder NH ist;
R8 aus der Gruppe bestehend aus H, Phenyl und 2-, 3-, 4-Pyridyl ausgewählt ist, wobei die letzten Vier wahlweise mit CHO, CN,
NO₂, CH₃ oder F substituiert sind;
HET2 aus der Gruppe bestehend aus: 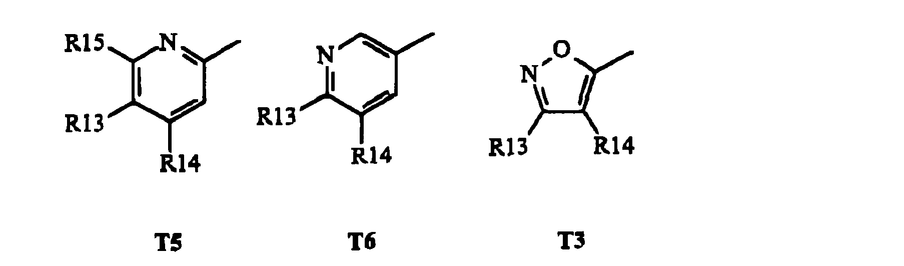 ausgewählt ist, worin R13, R14 und R15 unabhängig aus der Gruppe bestehend aus CN, *NO₂* und CHO ausgewählt sind;
und R6 aus der Gruppe bestehend aus H, CH₃, CN, CHO, CH₂OH, CH=N-OH, CH=CHCN, CO-CH₃ und CH₂NH-Phenyl ausgewählt ist, wobei dieses Phenyl mit einem Rest, ausgewählt aus der Gruppe bestehend aus F, CN, CHO und NO₂, substituiert ist.

3. Verbindung nach irgendeinem der Ansprüche 1 oder 2, zur Verwendung bei der therapeutischen Behandlung eines menschlichen oder tierischen Körpers.

4. Verbindung nach irgendeinem der Ansprüche 1 oder 2, zur Verwendung bei der Behandlung mikrobieller Infektionen.

5. Verbindung nach Anspruch 4, worin die Behandlung durch orale, parenterale oder topische Verabreichung durchgeführt wird.

6. Verwendung der in irgendeinem der Ansprüche 1 oder 2 definierten Verbindung zur Herstellung eines Medikaments zur Behandlung mikrobieller Infektionen.

7. Verwendung nach Anspruch 6, worin das Medikament auf dem oralen, parenteralen oder topischen Weg verabreicht werden kann.

8. Verbindung nach irgendeinem der Ansprüche 1 oder 2, zur Verwendung bei der Behandlung kanzeröser und präkanzeröser pathologischer Zustände.

9. Verbindung nach Anspruch 8, worin die Behandlung durch orale, parenterale oder topische Verabreichung durchgeführt wird.

10. Verwendung der in irgendeinem der Ansprüche 1 oder 2 definierten Verbindung zur Herstellung eines Medikaments zur Behandlung kanzer&ser oder präkanzeröser pathologischer Zustände.

11. Verwendung nach Anspruch 10, worin das Medikament auf dem oralen, parenteralen oder topischen weg verabreicht wird.

12. Pharmazeutische Zusammensetzung, umfassend eine therapeutisch wirksame Menge einer Verbindung, wie sie in irgendeinem der Ansprüche 1 oder 2 definiert ist, und pharmazeutisch annehmbare Exzipientien oder Lösungsmittel.

## Revendications

1. Composé du type isoxazole disubstitué en positions 3 et 5, de formule (1) : 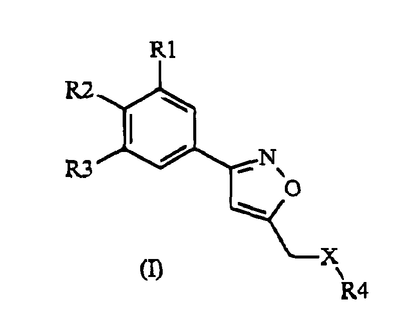 et les stéréoisomères, mélanges de stéréoisomères, formes polymorphes, mélanges de formes polymorphes, N-oxydes, produits de solvatation et sels pharmaceutiquement acceptables d'un tel composé, dans laquelle
X représente un radical divalent choisi parmi O, S, NH, OCO, NH-CO, NH-COO, NH-CS, NH-CO-NH et NH-CS-NH,
R4 est un radical choisi parmi un atome d'hydrogène,
un groupe alkyle en C₁ à C₃, éventuellement substitué par 1, 2 ou 3 atomes d'halogène pris parmi les atomes de fluor, de chlore et de brome, et
un radical hétérocyclique HET1, relié par un atome de carbone, qui est soit un radical, relié par un atome de carbone, d'un hétérocycle à 5 chaînons ayant 1, 2, 3 ou 4 hétéroatomes pris parmi N, O et S, et éventuellement substitué par un radical pris parmi les groupes alkyle en C₁ à C₄, le groupe amino, les groupes alkyl(C₁ à C₄)amino, les groupes alcoxy en C₁ à C₄, les groupes alcoxy(C₁ à C₄)carbonyle, les groupes alkyl(C₁ à C₄)carbonyle, les groupes amido en C₁ à C₄, le groupe amido, CN, NO₂, F, Cl et Br,
soit un radical, relié par un atome de carbone, d'un hétérocycle à 6 chaînons ayant 1, 2 ou 3 atomes d'azote et éventuellement substitué par 1, 2 ou 3 substituants pris indépendamment parmi les groupes alkyle en C₁ à C₄, le groupe amino, les groupes alkyl(C₁ à C₄)amino, les groupes alcoxy en C₁ à C₄, les groupes alcoxy(C₁ à C₄)carbonyle, les groupes alkyl(C₁ à C₄)carbonyle, les groupes amido en C₁ à C₄, le groupe amido, CN, NO₂, F, Cl et Br,
R1 et R3 représentent chacun indépendamment H ou F,
R2 représente un radical relié par un atome d'azote ou un atome de carbone, choisi parmi les radicaux suivants : 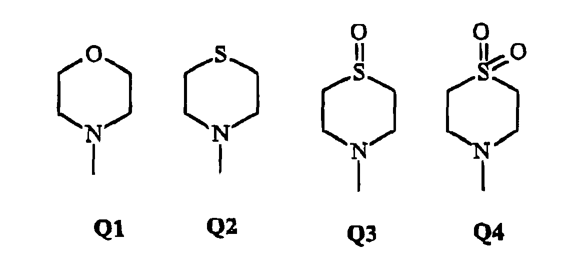 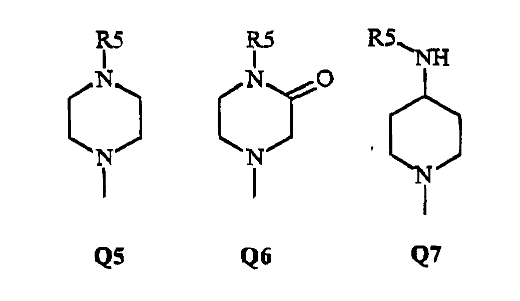 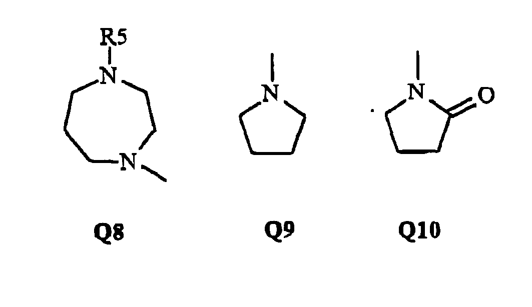 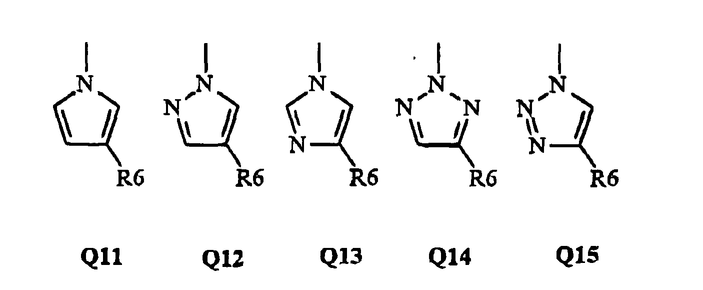 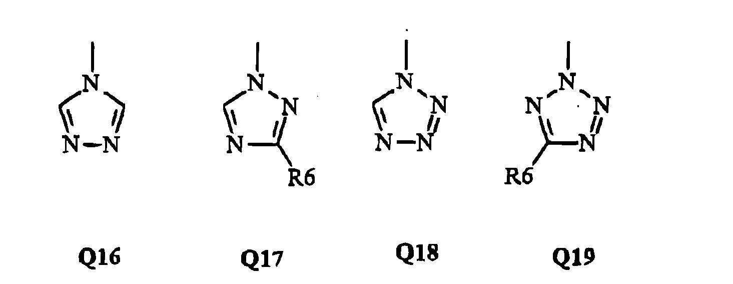 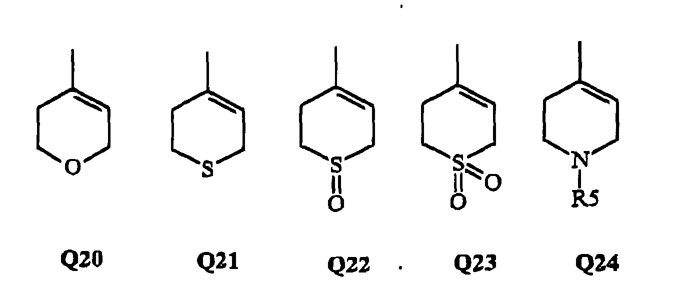 dans lesquels
R5 représente un radical non-cyclique, pris parmi les groupes -(CH₂)ₙ-CO-R7 et -SO₂-R7, où
R7 représente un groupe alkyle en C₁ à C₄, un groupe alcényle en C₁ à C₃, à chaîne droite ou ramifiée, un groupe -(CH₂)ₚ-R₂, un groupe - (CH₂)ₘ-Y-(CH₂)_{q}-R8 ou un radical HET2,
n, p, q et m représentent des nombres entiers de 0 à 8,
Y représente O, S ou NH,
R2 a les significations indiquées précédemment, à l'exclusion de Q20, Q21, Q22, Q23 et Q24,
R8 représente un atome d'hydrogène ou un radical, relié par un atome de carbone, choisi parmi les groupes alkyle en C₁ à C₃, vinyle, allyle, éthinyle, propargyle, phényle, phényle substitué par CHO, CN, NO₂, CH₃ ou F et les radicaux, reliés par un atome de carbone, des systèmes aromatiques formés par un cycle à 5 ou 6 chaînons ou par deux cycles condensés à 5 ou 6 chaînons, lesdits systèmes aromatiques contenant 1 à 3 hétéroatomes choisis indépendamment parmi O, N et S, et ledit système aromatique ou phényle étant éventuellement substitués par 1, 2 ou 3 radicaux choisis indépendamment parmi H, les groupes alkyle en C₁ à C₄, à chaîne droite ou ramifiée, les groupes alcoxy en C₁ à C₄, les groupes alkylsulfonyle en C₁ à C₄, NHCO-R9, NHCOO-R9, CO-R9, COO-R9, CN, NO, NO₂, CH=N-R10, F, Cl et Br ou ledit système aromatique étant choisi parmi les groupes 2-, 3- et 4-pyridyle, tous pouvant etre éventuellement substitués par CHO, CN, NO₂, CH₃ ou F ;
R9 désignant un atome d'hydrogène, un groupe alkyle en C₁ à C₃ ou un groupe N(R11)(R12) où R11 et R12 représentent chacun indépendamment un atome d'hydrogène ou un groupe alkyle en C₁ à C₃, et
R10 représentant un atome d'hydrogène ou un groupe alkyle en C₁ à C₃, phényle, benzyle, OH ou alcoxy en C₁ à C₃,
HET2 représente un radical hétérocyclique, relié par un atome de carbone, pris parmi les radicaux suivants : 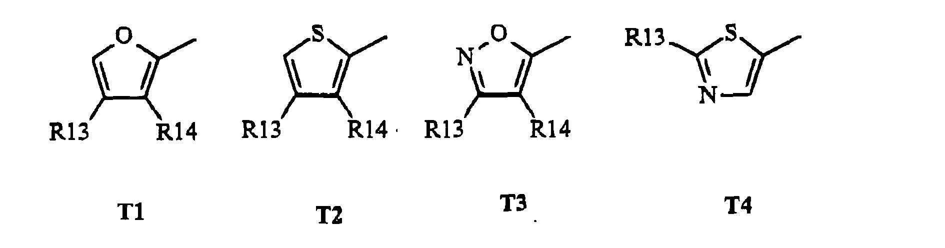 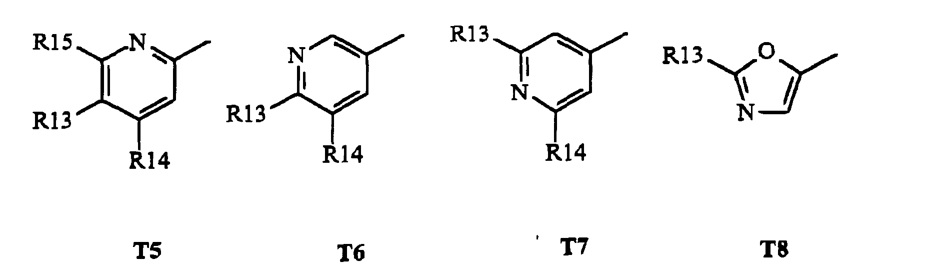 dans lesquels R13, R14 et R15 représentent des radicaux choisis indépendamment parmi H, les groupes alkyle en C₁ à C₄, à chaîne droite
ou ramifiée, les groupes alcoxy en C₁ à C₄, les groupes alkylsulfonyle en C₁ à C₄, NHCO-R9, NHCOO-R9, CO-R9, COO-R9, CN, NO, NO₂, CH=N-R10, F, Cl et Br, R9 et R10 étant tels que définis précédemment,
ou bien R5 représente un radical hétérocyclique relié par un atome de carbone, choisi parmi les radicaux : 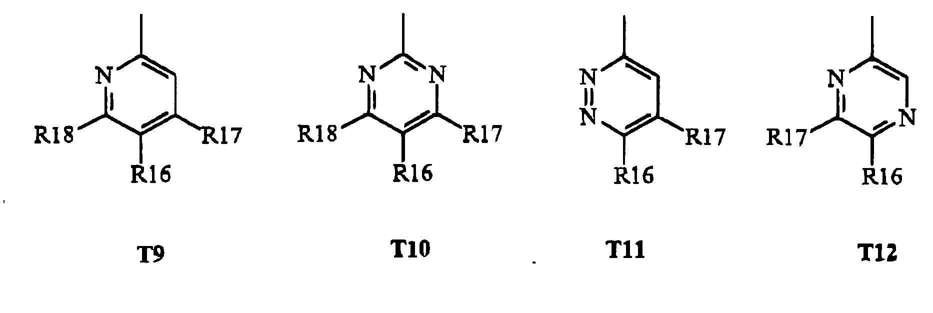 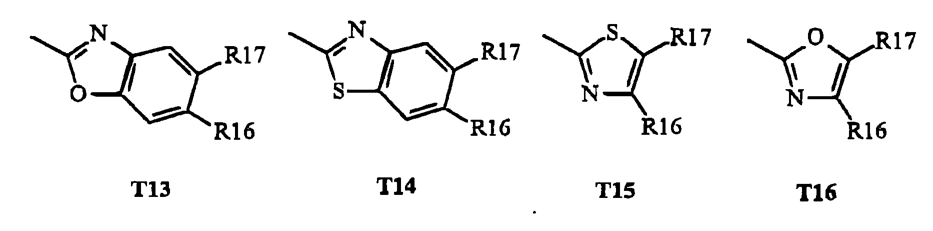 dans lesquels R16, R17 et R18 représentent chacun indépendamment COR9, COO-R9, CN, NO, NO₂ ou CH=N-R10,
R6 représente H, F, Cl, Br, un groupe trifluorométhyle, CN, NO₂, CHO, CH₂OH, un groupe alkyle en C₁ à C₃, un groupe alcoxy en C₁ à C₃, un groupe alcoxy(C₁ à C₃)carbonyle, un groupe alcoxy(C₁ à C₃)alkyle(C₁ à C₃), un groupe benzyloxyalkyle(C₁ à C₃), un groupe alkyl(C₁ à C₃)carbonyle, CO-NR19R20, NR19R20, un groupe alkyl(C₁ à C₃)amino, un groupe alkyl(C₁ à C₃)-CH=N-O-R21, CH=N.O-R21, CH=CR22R23, (CH₂)₈NHR₁₉ ou CH=NR19, R19 et R20 étant choisis indépendamment parmi H, les groupes alkyle en C₁ à C₃, CO-R24 et les systèmes aromatiques formés par un cycle à 5 ou 6 chaînons ou par deux cycles condensés à 5 ou 6 chaînons, ledit système aromatique contenant éventuellement 1 à 3 hétéroatomes pris indépendamment parmi O, N et S, et ledit système aromatique étant éventuellement substitué par 1, 2 ou 3 radicaux choisis indépendamment parmi H, les groupes alkyle en C₁ à C₄, à chaîne droite ou ramifiée, les groupes alcoxy en C₁ à C₄, les groupes alkylsulfonyle en C₁ à C₄, NHCO-R9, NHCOO-R9, CO-R9, COO-R9, CN, NO, NO₂, CH=N-R10, F, Cl et Br, R21 représentant H ou un groupe alkyle en C₁ à C₃, R22 et R23 représentant chacun indépendamment H, CN, NO₂, un groupe alkyl(C₁ à C₃)carbonyle, un groupe alcoxy(C₁ à C₃)carbonyle, CHO, CONR19R20 ou CH₂OH, R24 désignant H, un groupe alkyle en C₁ à C₃, un groupe alcoxy en C₁ à C₃ ou un radical HET2, HET2 étant tel que défini précédemment, et s désignant un nombre entier ayant une valeur de 0 à 4.

2. Composé selon la revendication 1, pour lequel :
X représente NH ou NH-CS,
R4 représente un groupe méthyle ou un radical isoxazolyle ou isothiazolyle, relié par un atome de carbone, éventuellement substitué par un groupe méthyle à l'une quelconque des positions substituables,
R1 représente H et R3 représente F,
R2 représente un radical pris parmi les radicaux suivants : 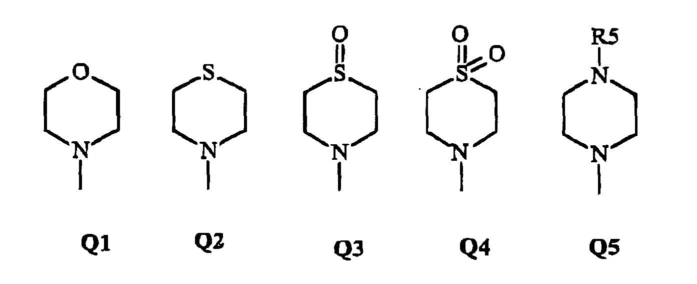 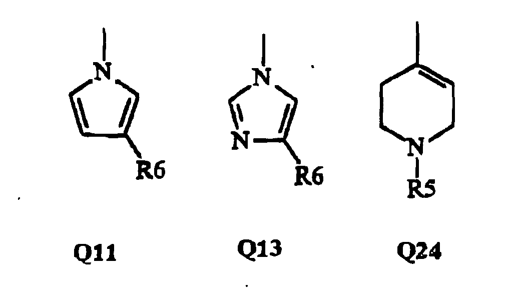
R5 représente CO-R7,
R7 représente (CH₂)ₘ-Y-R8 ou HET2, où m est égal à 1,
Y représente O ou NH,
R8 représente H, un groupe phényle ou un groupe 2-, 3- ou 4-pyridyle, ces quatre derniers groupes étant éventuellement substitués par CHO, CN, NO₂, CH₃ ou F, et
HET2 est un radical choisi parmi les radicaux suivants : 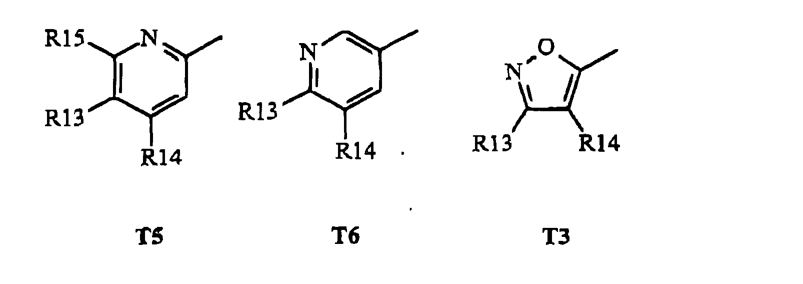 où R13, R14 et R15 représentent chacun indépendamment CN, NO₂ ou CHO,
et R6 représente H, CH₃, CN, CHO, CH₂OH, CH=N-OH, CH=CHCN, CO-CH₃ ou CH₂NH-phényl, ledit groupe phényle étant substitué par un radical pris parmi F, CN, CHO et NO₂.

3. Composé selon la revendication 1 ou 2, qui est destiné à être utilisé dans le traitement thérapeutique du corps humain ou du corps animal.

4. Composé selon la revendication 1 ou 2, qui est destiné à être utilisé dans le traitement d'infections microbiennes.

5. Composé selon la revendication 4, pour lequel le traitement est effectué par administration orale, parentérale ou topique.

6. Utilisation d'un composé tel que défini dans la revendication 1 ou 2, pour la préparation d'un médicament destiné au traitement d'infections microbiennes.

7. Utilisation selon la revendication 6, dans laquelle le médicament peut être administré par voie orale, parentérale ou topique.

8. Composé selon la revendication 1 ou 2, qui est destiné à être utilisé dans le traitement de pathologies cancéreuses ou précancéreuses.

9. Composé selon la revendication 8, pour lequel le traitement est effectué par administration orale, parentérale ou topique.

10. Utilisation d'un composé tel que défini dans la revendication 1 ou 2, pour la préparation d'un médicament destiné au traitement de pathologies cancéreuses ou précancéreuses.

11. Utilisation selon la revendication 10, dans laquelle le médicament est administré par voie orale, parentérale ou topique.

12. Composition pharmaceutique qui comprend une quantité thérapeutiquement efficace d'un composé tel que défini dans la revendication 1 ou 2, et des excipients ou solvants pharmaceutiquement acceptables.
